# FASCICULE DE BREVET EUROPEEN

(11) **EP 1 468 672 B1**
(45) Date de publication et mention de la délivrance du brevet: **20.08.2008**
(21) Numéro de dépôt: 04290649.5
(22) Date de dépôt: 10.03.2004
(51) Int. Cl.: A61K 8/06, A61K 8/37, A61Q 17/04

(54) **Emulsion photoprotectrice fine du type huile-dans-eau, son procédé de fabrication et son utilisation dans les domaines cosmétique et dermatologique**
Lichtschutzformulierung in der Form einer Öl-Wasser-Nanoemulsion, deren Herstellung und Verwendung in der Kosmetik und in der Dermatologie
Sunscreen oil-water nanoemulsion, its method of preparation and its uses in cosmetics and dermatology

(30) Priorité: 14.04.2003 FR 0304648
(43) Date de publication de la demande: 20.10.2004
(73) Titulaire: L'ORÉAL, 75008 Paris (FR)
(72) Inventeur: Simonnet, Jean-Thierry, 94240 Cachan (FR)
(74) Mandataire: Miszputen, Laurent

(56) Documents cités:
- EP-A- 0 864 320
- EP-A- 0 916 335
- EP-A- 1 008 586
- EP-A- 1 025 901
- EP-A- 1 172 077
- WO-A-02/080878
- FR-A- 2 801 210

## Description

L'invention se rapporte à une émulsion photoprotectrice huile-dans-eau dans laquelle les globules d'huile de l'émulsion ont un diamètre moyen d'au plus 500 nm contenant au moins des particules de polymère ionique et au moins un système filtrant les radiations UV, caractérisée par le fait que le système filtrant comprend au moins un filtre UV-A du type 4,4-diarylbutadiène.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et UVB.

Pour diverses raisons liées en particulier à leur grand confort d'utilisation et à leur fraîcheur, les compositions cosmétiques, et en particulier celles destinées à la photoprotection de la peau contre les rayons UV-A et UV-B, appelées compositions solaires, se présentent le plus souvent sous la forme d'une émulsion du type huile-dans-eau, comportant une phase huileuse dispersée de manière homogène dans une phase aqueuse. Dans ces émulsions classiques, qui contiennent des agents émulsionnants (ou tensioactifs) et d'éventuels additifs cosmétiques, la taille des globules constituant la phase grasse est généralement supérieure à plusieurs microns. De telles émulsions peuvent avoir des propriétés cosmétiques (toucher huileux) et physiques (stabilité) insuffisantes.

L'un des objectifs majeurs des compositions antisolaires de type H/E connues à ce jour est d'avoir une parfaite stabilité de l'émulsion associée à une photoprotection la plus large possible et une innocuité améliorée. Par stabilité de l'émulsion, il est entendu que la dispersion reste stable macroscopiquement et microscopiquement (granulométrie) sur une période de temps d'au moins 30j.

Par ailleurs, on observe que, malgré la présence des agents émulsionnants (ou tensioactifs), certaines de ces émulsions présentent un manque de stabilité dans le temps, manque de stabilité se traduisant par l'apparition d'un phénomène de séparation déphasage) entre les phases aqueuse et huileuse de l'émulsion. Cette instabilité nuit à la conservation des émulsions.

Aussi, pour éviter ce phénomène indésirable, il est souvent nécessaire de faire appel à des agents dits épaississants, que l'on introduit alors dans l'émulsion et dont la fonction première est de créer, au sein de la phase aqueuse, une matrice gélifiée servant à figer dans son réseau tridimensionnel les globules de la phase grasse, assurant ainsi un maintien mécanique de l'ensemble de l'émulsion. Toutefois, cette addition d'épaississant limite les formes galéniques possibles, en excluant notamment les compositions très fluides.

Or, on cherche de plus en plus à préparer des compositions fluides, notamment solaires, plus spécialement dans le but de disposer de produits facilement vaporisables souvent considérés par l'utilisateur comme plus faciles à appliquer que les crèmes.

Enfin, pour limiter au mieux les risques d'intolérance, notamment des peaux dites « sensibles », on cherche de plus en plus à limiter dans la fabrications des émulsions huile/eau au maximum l'usage de tensioactifs émulsionnants qui par leur action peuvent fragiliser la fonction barrière de l'épiderme.

Pour remédier à ces problèmes et répondre à ces besoins, on a mis au point dans la demande EP0864320 des émulsions fines et fluides particulièrement stables contenant et stabilisées par des particules de polymère ionique, les globules d'huile de ces émulsion ayant un diamètre moyen inférieur à 500 nanomètres. Ces émulsions présentent des qualités sensorielles (toucher) particulièrement satisfaisantes. Ces émulsions peuvent être utilisées pour la photoprotection de la peau et des cheveux contre les effets des rayons UV dans la mesure où elles peuvent contenir des filtres UV-A et ou des flitres UV-B.

Parmi les filtres UV-A organiques disponibles, une famille de composés particulièrement efficaces dans l'UV-A est l'acide benzène 1,4-di(3-méthylidène-10-camphosulfonique) et ses différents sels, décrite notamment dans les demandes de brevets FR-A-2528420 et FR-A-2639347, ils sont capables en effet d'absorber les rayons ultraviolets de longueur d'ondes comprises entre 280 et 400 nm, avec des maxima d'absorption compris entre 320 et 400 nm, en particulier aux alentours de 345 nm.

Cependant, l'introduction de ce type de filtre UVA même parfois à de faibles doses dans des émulsions huile-dans-eau fines stabilisées par des particules de polymère ionique conduit rapidement à leur déstabilisation. Ce qui oblige le formulateur à les utiliser à de très faibles concentrations et à limiter l'efficicté en photoprotection en particulier dans le domaine des UV-A.

Il apparaît ainsi nécessaire de disposer d'émulsions fines huile-dans-eau solaires à base des particules de polymère ionique qui soient stables et qui puissent contenir des filtres organiques actifs dans l'UV-A d'efficacité comparable à celle de l'acide benzène 1,4-di(3-méthylidène-10-camphosulfonique) et ses différents sels sans les inconvénients énumérés ci-dessus.

La Demanderesse a découvert de façon surprenante que les émulsions huile-dans-eau fines solaires à base des particules de polymère ionique et au moins un filtre UV-A du type 4,4-diarylbutadiène, répondaient à ce besoin.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Cette découverte est à la base de la présente invention.

Ainsi, conformément à l'un des objets de la présente invention, il est maintenant proposé une émulsion huile-dans-eau dans laquelle les globules d'huile de l'émulsion ont un diamètre moyen d'au plus 500 nm contenant au moins des particules de polymère ionique et au moins un système filtrant les radiations UV, caractérisée par le fait que le système filtrant comprend au moins un filtre UV-A du type 4,4-diarylbutadiène.

D'autres caractéristiques, aspects et avantages de l'invention apparaîtront à la lecture de la description détaillée qui va suivre.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

On entend par "polymère ionique" aussi bien un homopolymère qu'un copolymère. Les polymères ont notamment pour but de disperser la phase huileuse dans la phase aqueuse.

Les émulsions selon l'invention présentent notamment l'avantage de pouvoir être très fluides tout en présentant une très bonne stabilité, même en l'absence d'agent gélifiant.

En plus des avantages mentionnés ci-dessus (fluidité, stabilité), l'utilisation des particules de polymère comme dispersant permet d'effectuer à froid l'étape de dispersion de la phase huileuse dans la phase aqueuse, ce qui est plus simple et moins coûteux que les procédés classiques réalisés le plus souvent à chaud quand on utilise des tensioactifs. La fabrication à froid permet par exemple l'introduction d'actifs thermosensibles sans risque de dégradation de ces actifs.

Avantageusement, l'émulsion de l'invention est exempte de tensioactif. Ainsi, du fait de l'absence de tensioactif, cette émulsion présente l'avantage de ne pas être irritante pour les peaux particulièrement sensibles.

Par ailleurs, l'émulsion ainsi obtenue est très fine et présente des qualités sensorielles particulièrement satisfaisantes. La taille moyenne des globules constituant la phase huileuse est inférieure à 500 nm, et elle va de préférence de 150 nm à 300 nm.

L'émulsion selon l'invention peut être très fluide, ce qui signifie qu'elle peut présenter une viscosité inférieure 15 000 cPs (soit 15 Pa.s), encore plus préférentiellement inférieure à 5 000 cPs (soit 5 Pa.s) (mesurée sur viscosimètre Brookfield RVT modèle DV2, à 0,5 tour/mn et avec disque n°5).

De façon générale, les particules utilisables dans l'invention peuvent être réalisées à partir d'un polymère ionique, d'un mélange de polymères ioniques ou d'un mélange d'au moins un polymère ionique et d'au moins un polymère non-ionique. Ces polymères doivent être non toxiques et non irritants pour la peau. En outre, ils doivent pouvoir se disperser dans l'eau sous forme particulaire.

Le polymère ionique peut être cationique ou anionique.

II s'agit de préférence d'un polymère anionique.

Les polymères anioniques hydrodispersibles utilisables dans l'invention sont par exemple les polymères d'acide isophtalique ou d'acide sulfoisophtalique, et en particulier les copolymères de phtalate / sulfoisophtalate / glycol (par exemple diéthylèneglycol / Phtalate / isophtalate / 1,4-cyclohexane-diméthanol) vendus sous les dénominations "Eastman AQ polymer" (AQ35S, AQ38S, AQ55S, AQ48 Ultra) par la société Eastman Chemical.

Ces polyesters peuvent aussi contenir des motifs dérivés d'éthylèneglycol, de triéthylèneglycol et/ou de tétraéthylèneglycol et d'acide téréphtalique comme ceux commercialisés sous les dénominations POLYCARE PS 20, POLYCARE PS30 et POLYCARE PS 32 par la société Rhodia.

La proportion de motifs dérivés d'acide sulfoisophtalique varie de préférence de 2 à 20 % en poids par rapport au poids total du polymère.

Les polymères anioniques hydrodispersibles utilisables dans l'invention peuvent être également parmi les copolymères vinyliques filmogènes utilisés couramment pour la préparation de compositions cosmétiques parm lesquels on peut citer :
(i) les copolymères acétate de vinyle/acide crotonique polyéthoxylés tels que celui commercialisé sous la dénomination ARISTOFLEX A par la société HOECHST ;
(ii) les copolymères acétate de vinyle/acide crotonique tels que celui commercialisé sous la dénomination LUVISET CA66 par la société BASF ;
(iii) les terpolymères acétate de vinyle/acide crotonique/néodécanoate de vinyle tels que celui commercialisé sous la dénomination RÉSINE 28-29-30, par la société NATIONAL STARCH ;
(iv) les copolymères N-octylacrylamide/méthacrylate de méthyle/méthacrylate d'hydroxypropyle/acide acrylique/méthacrylate de tert-butylaminoéthyle tels que celui commercialisé sous la dénomination de AMPHOMER par la société NATIONAL STARCH.
(v) les copolymères alternés méthylvinyléther/anhydride maléique monoestérifiés par le butanol, tels que celui commercialisé sous la dénomination GANTREZ ES 425 par la société GAF ;
(vi) les terpolymères acide acrylique/acrylate d'éthyle/N-tert-butylacrylamide tels que celui commercialisé sous la dénomination ULTRAHOLD 8 par la société BASF.

Comme polymères anioniques hydrodispersibles naturels susceptibles d'être utilisés selon la présente invention, on peut citer la résine shellac, la gomme de sandaraque et les dammars.

La résine shellac est une sécrétion animale, composée principalement de résine et de cire et est soluble dans certains solvants organiques. Elle doit être sous-neutralisée pour ne pas devenir soluble dans l'eau.

Les dammars sont des résines provenant d'arbres des genres Damara ou Shoréa et contiennent généralement 62,5 % de résènes (40 % de solubles et 22,5% d'insolubles dans l'alcool) et 23 % d'acides.

La masse molaire moyenne en poids des polymères anioniques hydrodispersibles de la présente invention est généralement allant de 1000 à 5 000 000.

De façon avantageuse, les particules de polymère ionique utilisées selon l'invention ont une granulométrie allant de 10 à 400 nm et encore mieux allant de 20 à 200 nm selon la nature du polymère ionique.

Les particules de ces polymères peuvent être utilisées telles quelles ou en dispersion dans l'eau.

Le rapport entre la quantité de particules de polymère et la quantité de phase huileuse va de 1/5 à 1/40.

Dans les émulsions de l'invention, on peut utiliser une quantité de particules de polymère allant de 0,1 à 10 %, de préférence de 0,5 à 5 % et mieux de 1 à 2 % du poids total de la composition.

A ces émulsions, est associé un procédé particulier par homogénéisation haute pression permettant d'obtenir la taille requise. La réquisition de cette taille s'expliquant par une amélioration très sensible de la stabilité de la dispersion.

Une première étape consiste à mélanger sous agitation la phase aqueuse, la phase huileuse et les particules de polymère et, dans une seconde étape à soumettre le mélange obtenu à une homogénéisation basée sur le principe de la cavitation.

Le mélange est tout d'abord soumis à une agitation classique par exemple dans un homogénéisateur tournant à une vitesse comprise entre 500 et 5000 tours/mn pendant un temps compris entre 10 et 60 mn environ à une température comprise entre 20 et 95°C environ.

L'homogénéisation basée sur le principe de la cavitation de la deuxième étape est une étape clé du procédé selon l'invention. Cette homogénéisation résulte du phénomène de cavitation créé et entretenu au sein du mélange, alors sous forme liquide, en mouvement à une vitesse linéaire d'au moins 100m/s.

Cette homogénéisation peut être opérée par utilisation d'un homogénéisateur haute pression fonctionnant sous une pression allant de 100 à 1000 bars environ, et de préférence de 400 à 700 bars. Le principe d'utilisation de ce type d'homogénéisateur est bien connu de l'homme de l'art. On opère à la température ambiante par passages successifs, généralement de 2 à 10 passages, sous la pression retenue, le mélange étant ramené à la température ambiante entre chaque passage.

L'homogénéisation peut également être obtenue sous l'action d'ultrasons ou encore par utilisation d'homogénéisateurs équipés d'une tête de type rotor-stator.

Les composés 4,4-diarylbutadiènes conformes à l'invention sont choisis de préférence parmi ceux répondant à la formule (I) suivante : 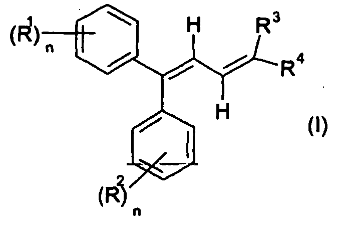 dans laquelle le système diène est de configuration Z,Z ; Z,E ; E,Z ou E,E ou des mélanges desdites configurations et où :
- R¹ et R², identiques ou différents, désignent hydrogène, un radical alkyle en C₁-C₂₀,; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ ; un radical monoalkylamino en C₁-C₁₂ ; un radical dialkylamino en C₁-C₁₂ ; un aryle ; un hétéroaryle
ou un substituant hydrosolubilisant choisi parmi un reste carboxylate, sulfonate ou un reste ammonium ;
- R³ désigne un groupe COOR⁵ ; COR⁵ ; CONR⁵R⁶ ; CN ; O=S(-R⁵)=O ; O=S(-OR5)=O ; R⁷O-P-(-OR⁸)=O; un radical alkyle en C₁-C₂₀; un radical alcényle en C₂- C₁₀ ; un radical cycloalkyle en C₃-C₁₀ ; un radical bicycloalkyle en C₇-C₁₀; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- R⁴ désigne un groupe COOR⁶; COR⁶ ; CONR⁵R⁶ ; CN ; O=S(-R⁶)=O ; O=S(-OR⁶)=O ; R⁷C-P-(-OR⁸)=O; un radical alkyle en C₁-C₂₀; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- les radicaux R⁵ à R⁸, identiques ou différents, désignent hydrogène ; un radical alkyle en C₁-C₂₀ ; un radical alcényle en C₂-C₁₀; un radical cycloalkyle en C₃-C₁₀; un radical bicycloalkyle en C₇-C₁₀ ; un radical bicycloalcényle en C₃-C₁₀ ; un radical cycloalcényle en C₇-C₁₀ ; un aryle éventuellement substitué ; un hétéroaryle éventuellement substitué ;
- n varie de 1 à 3 ;
les radicaux R³ à R⁸ peuvent former entre eux avec les atomes de carbone auxquels ils sont liés, un noyau en C₅-C₆ pouvant être condensé.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : éthènyle, n-propènyle, 1-méthyléthènyle, n-butènyle, 1-méthylpropènyle, 2-méthylpropènyle, 1,1-diméthyléthènyle, n-pentènyle, 1-méthylbutènyle, 2-méthylbutènyle, 3-méthylbutènyle, 2,2-diméthylpropènyle, 1-éthylpropènyle, n-hexènyle, 1,1-diméthylpropènyle, 1,2-diméthylpropènyle, 1-méthylpentènyle, 2-méthylpentènyle, 3-méthylpentènyle, 4-méthylpentènyle, 1,1-diméthylbutènyle, 1,2-diméthylbutènyle, 1,3-diméthylbutènyle, 2,2-diméthylbutènyle, 2,3-diméthylbutènyle, 3,3-diméthylbutènyle, 1-éthylbutènyle, 2-éthylbutènyle, 1,1,2-triméthytpropènyle, 1,2,2-triméthylpropènyle, 1-éthyl-1-méthylpropènyle, 1-éthyl-2-méthylpropènyle, n-heptènyle, n-octènyle, n-nonènyle, n-décènyle.

Comme radicaux alcoxy en C₁-C₁₂, on peut citer : méthoxy, n-propoxy, 1-méthylpropoxy, 1-méthyléthoxy, n-pentoxy, 3-méthylbutoxy, 2,2-diméthylpropoxy, 1-méthyl-1-éthylpropoxy, octoxy, éthoxy, n-propoxy, n-butoxy, 2-méthylpropoxy, 1,1-diméthylpropoxy, hexoxy, heptoxy, 2-éthylhexoxy.

Comme radicaux alcoxycarbonyle en C₁-C₂₀, on peut citer les esters des alcools en C₁-C₂₀.

Comme radicaux monoalkylamino ou dialkylamino en C₁-C₁₂, on peut citer ceux dont le ou les radicaux alkyle sont choisis parmi méthyle, n-propyle, 2-méthylpropyle, 1,1-diméthyléthyle, hexyle, heptyle, 2-éthylhexyle, isopropyle, 1-méthylpropyle, n-pentyle, 3-méthylbutyle, 2,2-diméthylpropyle, 1-méthyl-1-éthylpropyle, octyle.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctétraènyle, cyclononènyle ou cyclodécènyle.

Les radicaux cycloalkyles ou cycloalcényles peuvent comporter un ou plusieurs substituants (de préférence de 1 à 3) choisis par exemple parmi les halogènes comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres peuvent être saturées par un hydrogène ou un radical alkyle en C₁-C₄.

Les groupes bicycloalkyles ou bicycloalcényles sont choisis par exemple parmi les terpènes bicycliques comme les dérivés de pinane, de bornane, de pinène ou de camphre ou d'adamantane.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy. On préfère plus particulièrement phényle, méthoxyphényle , naphtyle, thienyle.

Les groupes hétéroaryles comportent en général un ou plusieurs hétéroatomes choisis parmi souffre, oxygène ou azote.

Les groupes hydrosolubilisants sont par exemple des restes carboxy, sulfoxy et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Les composés de formule (I) sont connus en eux-mêmes et leurs structures et leurs synthèses sont décrites dans les demandes de brevet DE19755649 , EP916335, EP1133980 et EP1133981.

### A titre d'exemple de composé de formule (I), on peut citer les composés suivants :

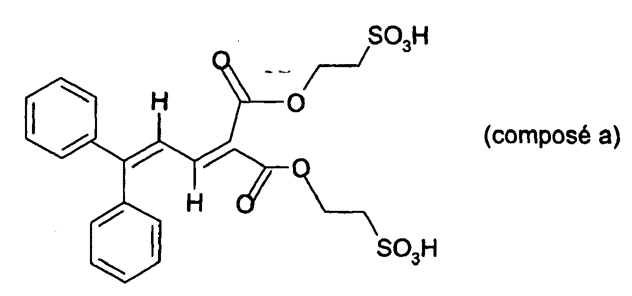

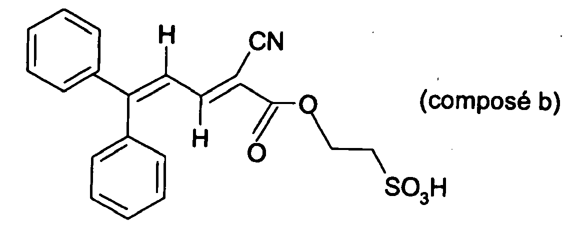

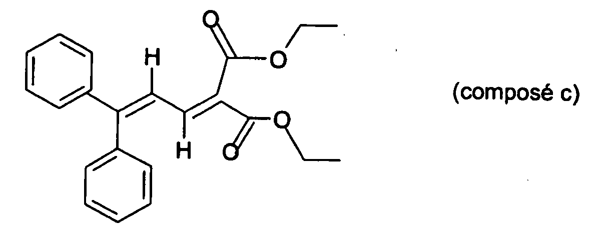

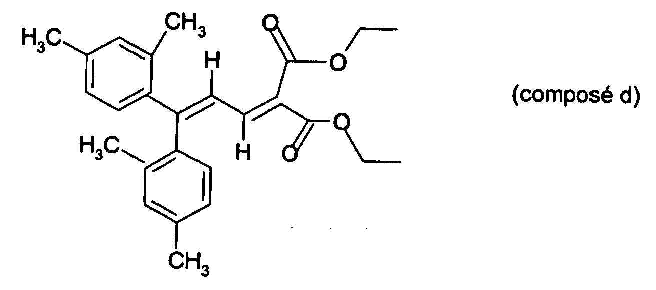

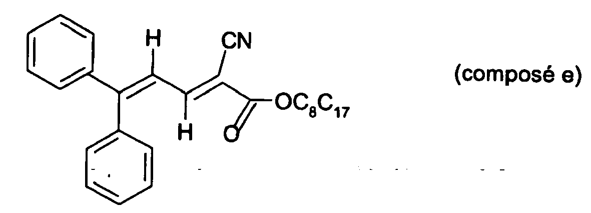

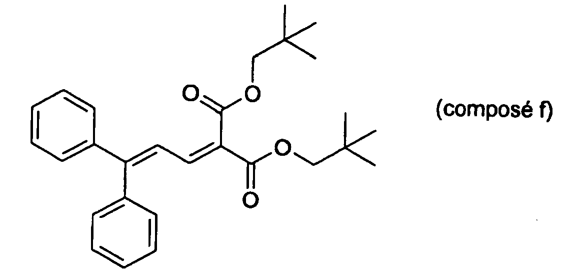

Les composés de formule (1) préférentiels sont ceux pour lesquels
- n = 1 ou 2 ;
- R¹ et R², identiques ou différents, désignent hydrogène, un radical alkyle en C₁-C₂₀,; un radical alcoxy en C₁-C₁₂ ; un radical monoalkylamino en C₁-C₁₂ ; un radical dialkylamino en C₁-C₁₂ ; un substituant hydrosolubilisant choisi parmi un groupe carboxylate, un groupe sulfonate ou un reste ammonium ;
- R³ désigne un groupe COOR⁵ ; COR⁵ ; CONR⁵R⁶ ; un radical alkyle en C₁-C₂₀ ; un radical cycloalkyle en C₃-C₁₀ ; un radical cycloalcényle en C₃-C₁₀ un radical bicycloalkyle en C₇-C₁₀ ; phényle, naphtyle ou thienyle éventuellement substitué ;
- R⁴ désigne un groupe COOR⁶ ; COR⁶ ; CONR⁵R⁶ ; un radical alkyle en C₁-C₂₀ ; un radical cycloalkyle en C₃-C₆ ; un radical cycloalcényle en C₃-C₁₀ ; un radical bicycloalkyle en C₇-C₁₀ ; phényle, naphtyle ou thienyle éventuellement substitué ;
- les radicaux R⁵ et R⁶, identiques ou différents, désignent hydrogène ; un radical alkyle en C₁-C₁₂ ; un radical cycloalkyle en C₃-C₁₀ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.
   Parmi ces composés, on préfère plus particulièrement ceux pour lesquels
- R¹ et R², identiques ou différents, désignent hydrogène, un radical alkyle en C₁-C₂₀ ; un radical alcoxy en C₁-C₂₀ ; un substituant hydrosolubilisant choisi parmi un groupe carboxylate, un groupe sulfonate ou un reste ammonium ;
- R³ désigne un groupe COOR⁵ ; COR⁵ ; CONR⁵R⁶ ;
- R⁴ désigne un groupe COOR⁶ ; COR⁶ ; CONR⁵R⁶ ;
- les radicaux R⁵ et R⁶, identiques ou différents, désignent hydrogène ; un radical alkyle en C₁-C₁₂ ; un radical cycloalkyle en C₃-C₆ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.

Selon un mode particulièrement préféré, les composés de formule (1) sont choisis parmi ceux de formule (I') suivante : 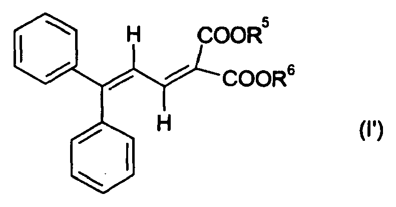 où les radicaux R⁵ et R⁶, identiques ou différents, désignent hydrogène ; un radical alkyle en C₁-C₂₀; un radical cycloalkyle en C₃-C₆ ; un radical cycloalcényle en C₃-C₁₀.

Parmi ces composés de formule (I'), on retient plus particulièrement le 1,1-dicarboxy-(2'2'-diméthyl-propyl)-4,4-diphénylbutadiène de structure :

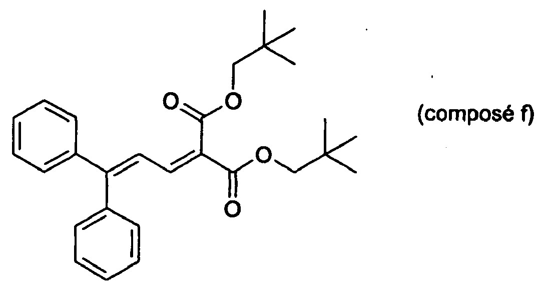

Une autre famille de 4,4-diarylbutadiène pouvant être utilisée dans les émulsions selon l'invention sont ceux répondant à la formule (II) suivante : 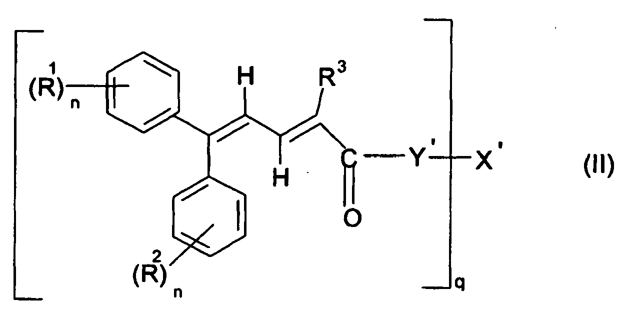 dans laquelle le système diène est de configuration Z,Z ; Z,E ; E,Z ou E,E ou des mélanges desdites configurations et où :
- R¹, R², R³ et n ont les mêmes significations indiquées dans la formule (I) précédente ;
- Y' désigne un groupe -O- ou -NR⁹-
- R⁹ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀; un radical cycloalcényle en C₃-C₁₀; ; un radical bicycloalcényle en C₇-C₁₀ ; un aryle ; un hétéroaryle;
- X' désigne un reste de polyol en C₂-C₂₀ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

X' est un reste polyol en C₂-C₂₀ contenant de 2 à 10 groupes hydroxyles et notamment :

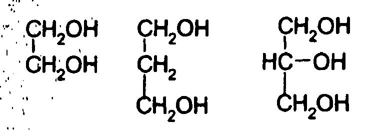

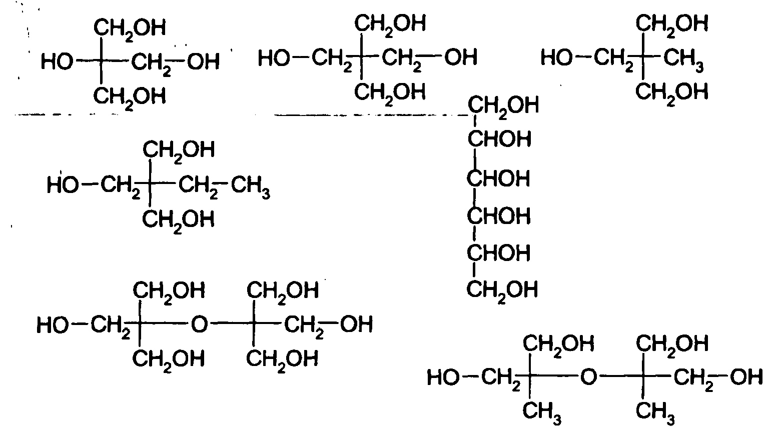

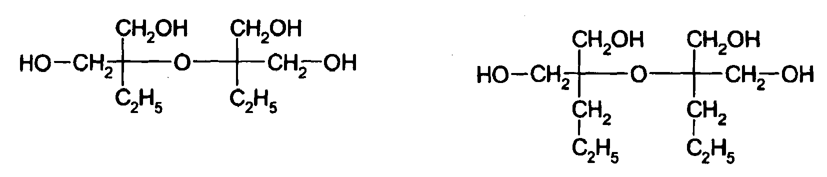

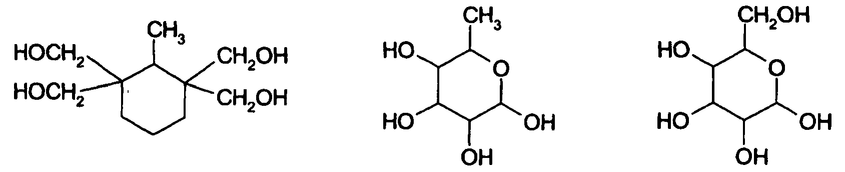

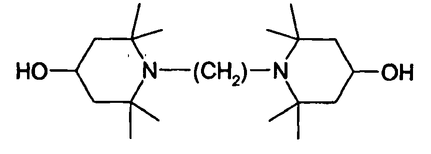

Les composés plus préférentiels de formule (II) sont ceux pour lesquels :
- R¹ et R², identiques ou différents, désignent hydrogène, un radical alkyle en C₁-C₁₂ ; un radical alcoxy en C₁-C₈ ; un substituant hydrosolubilisant choisi parmi un groupe carboxylate, un groupe sulfonate ou un reste ammonium ;
- R³ désigne un groupe COOR⁵ ; CONR⁵R⁶ ; CN ; un radical cycloalkyle en C₃-C₁₀ ; un radical bicycloalkyle en C₇-C₁₀ ;
- R⁵ et R⁶ , identiques ou différents, désignent un radical alkyle en C₁-C₂₀, linéaire ou ramifié ; un radical cycloalkyle en C₃-C₁₀ ; un radical bicycloalkyle en C₇-C₁₀; naphtyle ou phényle éventuellement substitué ;
- X' désigne un reste de polyol en C₂-C₂₀ comprenant de 2 à 6 groupes hydroxy et plus particulièrement de 2 à 4.

Les composés encore plus préférentiels de formule (II) sont ceux pour lesquels :
- X' désigne un reste d'éthanol ou de pentaerythritol.

Les composés de formule (II) encore plus particulièrement préférés sont choisis parmi

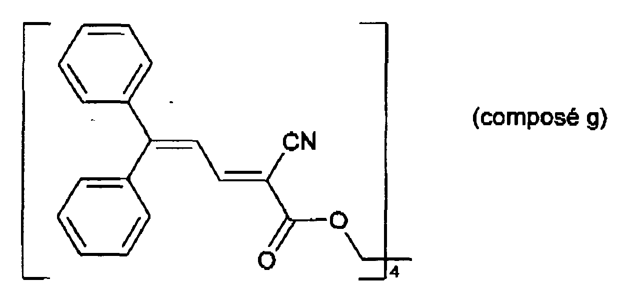

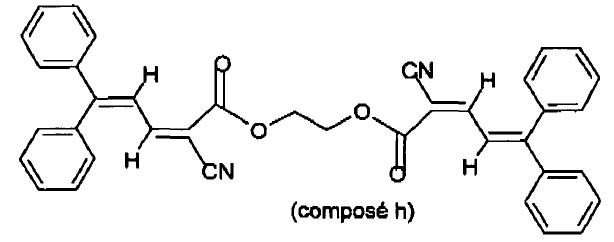

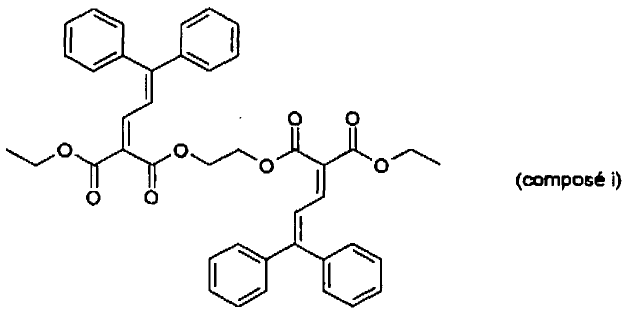

Les composés de formule (II) tels que définis ci-dessus sont connus en eux-mêmes et leurs structures et leurs synthèses sont décrites dans la demande de brevet EP-A-1008586.

Les composés 4,4-diarylbutadiène sont présents de préférence dans la composition dans des proportions allant de 0,1 % à 20% en poids, plus préférentiellement de 1 à 10% en poids par rapport au poids total de la composition.

Selon un mode particulier de réalisation de l'invention, les émulsions huile-dans-eau ou eau-dans huile préparées avec les particules de polymère ionique selon l'invention peuvent comporter seulement 1% en poids ou moins, et même être exemptes de tensioactifs émulsionnants, tout en étant stables au stockage.

La nature de la phase grasse rentrant dans la composition des émulsions selon l'invention n'est pas critique et elle peut ainsi être constituée par tous les composés qui sont déjà connus de façon générale comme convenant pour la fabrication d'émulsions de type eau dans huile. En particulier, ces composés peuvent être choisis, seuls ou en mélanges, parmi les différents corps gras, les huiles d'origine végétale, animale ou minérale, les cires naturelles ou synthétiques, et analogues. Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-a-oléfines.

Comme autres huiles utilisables dans les émulsions selon l'invention, on peut encore citer les benzoates d'alcools gras en C12-C15 (Finsolv TN de FINETEX), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C₁₀-C₁₈, les huiles fluorées et perfluorées, la lanoline, la lanoline hydrogénée, la lanoline acétylée et enfin les huiles de silicones, volatiles ou non.

La phase huileuse de l'émulsion peut représenter de 0,1 à 45 % et mieux de 5 à 30 % du poids total de l'émulsion.

Bien entendu, la phase grasse peut également contenir un ou plusieurs adjuvants cosmétiques lipophiles classiques, notamment ceux qui sont déjà utilisés de manière habituelle dans la fabrication et l'obtention des compositions cosmétiques antisolaires.

De manière classique, la phase aqueuse dispersante peut être constituée par de l'eau,
ou un mélange d'eau et d'alcool(s) polyhydrique(s) comme par exemple glycérol, propylèneglycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvents cosmétiques classiques hydrosolubles.

Les compositions conformes à l'invention peuvent comporter en plus d'autres filtres UV organiques ou inorganiques complémentaires actifs dans l'UVA et/ou l'UVB, hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les dérivés de méthylène bis-(hydroxyphényl benzotriazole) tels que décrits dans s les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 et leurs mélanges.

Comme exemples de filtres organiques actifs dans l'UV-A et/ou l'UV-B, on peut citer désignés ci-dessus sous leur nom INCI :
Dérivés de l'acide para-aminobenzoique :
   PABA,
   Ethyl PABA,
   Ethyl Dihydroxypropyl PABA,
   Ethylhexyl Diméthyl PABA vendu notamment sous le nom « ESCALOL 507 » par ISP, Glyceryl PABA,
   PEG-25 PABA vendu sous le nom « UVINUL P25 » par BASF,
Dérivés salicyliques:
   Homosalate vendu sous le nom « Eusolex HMS » par Rona/EM Industries,
   Ethylhexyl Salicylate vendu sous le nom « NEO HELIOPAN OS » par Haarmann et REIMER,
   Dipropyleneglycol Salicylate vendu sous le nom « DIPSAL » par SCHER,
   TEA Salicylate, vendu sous le nom « NEO HELIOPAN TS » par Haarmann et REIMER,
Dérivés du dibenzoylméthane :
   Butyl Methoxydibenzoylmethane vendu notamment sous le nom commercial « PARSOL 1789 » par HOFFMANN LAROCHE,
   Isopropyl Dibenzoylmethane,
Dérivés cinnamiques:
   Ethylhexyl Methoxycinnamate vendu notamment sous le nom commercial « PARSOL MCX » par HOFFMANN LAROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   - Diisopropyl Methylcinnamate,
   Glyceryl Ethylhexanoate Dimethoxycinnamate
Dérivés de β,β-diphénylacrylate :
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF, Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF, Benzophenone-12,
   le 2-(4-diéthylamino-2-hydroxybenzoyl)-benzoate de n-hexyle
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK ,
   Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
   - Terephthalylidene Dicamphor Sulfonic Acid fabriqué sous le nom « MEXORYL SX » par CHIMEX,
   Polyacrylamidomethyl Benzylidene Camphor fabriqué sous le nom « MESORYL SW » par CHIMEX,
Dérivés de benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par Haarmann et REIMER,
Dérivés de triazine :
   Anisotriazine vendu sous le nom commercial «TINOSORB S » par CIBA SPECIALTY CHEMICALS
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V
   la 2,4,6- tris-(4'amino-benzalmalonate de diisobutyle)-s-triazine.
Dérivés de benzotriazole :
   Drometrizole Trisiloxane vendu sous le nom « Silatrizole » par RHODIA CHIMIE ,
   Methylène bis-Benzotriazolyl Tetramethylbutylphénol, vendu sous forme solide sous le nom commercial « MIXXIM BB/100» par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par Haarmann et REIMER,
Dérivés d'imidazolines :
   Ethylhexyl Dimethoxybenzylidene Dioxoimidazoline Propionate,
Dérivés de benzalmalonate :
   Polyorganosiloxane à fonctions benzalmalonate tel que le polysilicone-15 vendu sous la dénomination commerciale « PARSOL SLX » par HOFFMANN LAROCHE.
Dérivés de benzoxazole :
   2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ;
   et leurs mélanges.

Les filtres organiques plus particulièrement préférés sont choisis parmi les composés suivants :
Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle 4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone 15
2,4-bis-[5-1 (diméthylpropyl)benzoxazol-2-yl-(4-phenyl)-imino]-6-(2-ethylhexyl)-imino-1,3,5-triazine et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres complémentaires selon l'invention sont généralement présents dans les compositions selon l'invention à une teneur allant de 0,1 % à 30 % en poids et de préférence de 0,5 à 15 % , en poids, par rapport au poids total de la composition.

Les compositions selon l'invention peuvent également contenir des agents de bronzage et/ou de brunissage artificiels de la peau (agents autobronzants).

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Les compositions conformes à la présente invention peuvent comprendre en outre des adjuvants cosmétiques classiques notamment choisis parmi les solvants organiques, les épaississants et/ou agents gélifiants ioniques ou non ioniques, les adoucissants, les humectants, les opacifiants, les stabilisants, les émollients, les silicones, les agents répulsifs contre les insectes, les parfums, les conservateurs, les tensioactifs, les charges, les pigments, les polymères, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels composés complémentaires cités ci-dessus et/ou leurs quantités de manière telle que les propriétés avantageuses attachées intrinsèquement aux émulsions conformes à l'invention ne soient pas, ou substantiellement pas, altérées par la ou les adjonctions envisagées.

Parmi les solvants organiques, on peut citer les alcools et polyols inférieurs.

Si l'on souhaite obtenir une émulsion moins fluide et/ou améliorer son toucher cosmétiques, on peut y ajouter un ou plusieurs gélifiants. Ces gélifiants sont utilisés à des concentrations allant de 0,1 à 10 %, de préférence 0,1 à 5 % et mieux de 0,1 à 3 % du poids total de la composition.

A titre de gélifiants, on peut citer les polymères associatifs non-ioniques ou anioniques hydrosolubles ou hydrodispersibles ayant des propriétés amphiphiles comportant au moins une séquence hydrophobe et au moins une séquence hydrophile.

### Polymères non-ioniques associatifs hydrosolubles ou hydrodispersibles,

La ou les séquences hydrophobes sont principalement des chaînes grasses ayant de 6 à 30 atomes de carbone, notamment hydrocarbonées telles que alkyle, arylalkyle(C₁-C₈), alkyl(C₁-C₈)aryle, alcényle, des groupements divalents aliphatiques tels que alkylène en C6-C30, des groupements divalents cycloaliphatiques tels que notamment méthylènedicyclohexyl, isophorone ou des groupements divalents aromatiques tels que phénylène. Les radicaux aryle désignent de préférence des groupements phényle, naphtyle ou anthryle.

La ou les séquences hydrophiles, peuvent être, entre autre, un polyoxyde d'éthylène, un polysaccharide, un polyamide notamment polyacrylamide, un polyester et leurs mélanges et de préférence un polyoxyde d'éthylène ayant de 15 à 500 oxydes d'éthylène. La ou les liaisons entre séquence hydrophobe et hydrophile est le plus souvent, sans être limitative, de type ester, éther, urée, amide ou uréthane et leurs mélanges.

Le rapport (en poids) de la (ou les) séquence(s) hydrophile(s) sur la (ou les) séquence(s) hydrophobe(s) du polymère, est de préférence compris entre 10/1 et 1000/1.

Les polymères non-ioniques associatifs hydrosolubles ou hydrodispersibles sont choisis de préférence parmi :
**(1)** Les celluloses modifiées par des groupements comportant au moins une chaîne hydrophobe ;
   on peut citer à titre d'exemple :
   - les hydroxyéthylcelluloses modifiées par des groupements comportant au moins une chaîne grasse tels que des groupes alkyle, arylalkyle, alkylaryle, alcényle ou leurs mélanges, et dans lesquels les groupes alkyle ou alcényle sont de préférence en C₈ -C₂₂ , comme le produit NATROSOL PLUS GRADE 330 CS (al-kyles en C 16 ) vendu par la société AQUALON, ou le produit BERMOCOLL EHM100 vendu par la société BEROL NOBEL,
   - celles modifiées par des groupes polyalkylène glycol éther d'alkyl (C6-C20)phénol, tel que le produit AMERCELL POLYMER HM-1500 (polyéthylène glycol(15) éther de nonyl phénol) vendu par la société AMERCHOL.
**(2)** Les hydroxypropylguars modifiés par des groupements comportant au moins une chaîne grasse en C10-C30 tel que le produit ESAFLOR HM 22 (chaîne alkyle en C₂₂ ) vendu par la société LAMBERTI, les produits MIRACARE XC95-3 (chaîne alkyle en C₁₄ ) et RE205-1 (chaîne alkyle en C₂₀ ) vendus par la société RHODIA CHIMIE.
**(3)** Les uréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaî-nements cycloaliphatiques et/ou aromatiques.
   De préférence, les polyéthers polyuréthanes comportent au moins deux chaînes lipophiles hydrocarbonées, ayant de C6 à C30 atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter une chaîne hydrocarbonée à un bout ou aux deux bouts d'une séquence hydrophile.
   Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
   De préférence, les polyéthers polyuréthanes non-ioniques sont des copolymères triblocs dont la séquence hydrophile comporte au moins une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
   Par extension figurent aussi parmi les polyéthers polyuréthanes non-ioniques, ceux dont les séquences hydrophiles sont liées par d'autres liaisons chimiques aux séquences lipophiles.
   A titre d'exemples de polyéthers polyuréthanes non-ioniques associatifs utilisables dans l'invention, on peut citer le polymère SER-AD FX1100 vendu par la société SERVO DELDEN, molécule comportant un motif oxyéthyléné et deux groupes hydrocarbonés en C₁₈ en bout de chaîne reliés à l'oxyde d'éthylène par l'intermédiaire d'une séquence polyuréthane. Comme polymère, on peut aussi utiliser aussi le Rhéolate 205 à fonction urée vendu par la société RHEOX ou encore le Rhéolate 208 , 204 ou 212 ; ainsi que l'Acrysol RM 184 de la société ROHM & HAAS ;
**(4)** Les copolymères de vinyl pyrrolidone et de monomères hydrophobes à chaîne grasse ; on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone/ eicosène) vendu par la société I.S.P.
**(5)** Les copolymères de méthacrylates ou d'acrylates d'alkyles en C₁-C₆ et de monomères amphiphiles comportant au moins une chaîne grasse tels que par exemple le copolymère méthacrylate de méthyle/acrylate de stéaryle oxyéthyléné vendu par la société GOLDSCHMIDT sous la dénomination ANTIL 208.
**(6)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

### Polymères anioniques associatifs

Ils sont en général solubles dans l'eau à un pH supérieur à 3,5. Ils comportent au moins une chaîne hydrophobe, ils sont non réticulés et ils ont de préférence un poids moléculaire allant de 10.000 à 2.000.000. Ces polymères permettent d'augmenter la viscosité des émulsions fluides (5 cP) d'au moins d'un facteur 10.

La ou les chaînes hydrophobes des polymères anioniques associatifs utilisés selon l'invention sont notamment des chaînes hydrocarbonées linéaires ou ramifiées, saturées
ou insaturées, ayant de 6 à 30 atomes de carbone, telles que alkyle, arylalkyle, alkylaryle, alcoylène ; des groupements divalents cycloaliphatiques tels que notamment méthylènedicyclohexyl et isophorone ; ou des groupements divalents aromatiques tels que phénylène.

Les polymères anioniques utilisés dans la présente invention sont choisis de préférence parmi les copolymères d'acide acrylique ou méthacrylique comportant une séquence hydrophile et au moins une séquence hydrophobe.

On entend par « copolymères » aussi bien les copolymères obtenus à partir de deux sortes de monomères que ceux obtenus à partir de plus de deux sortes de monomères tels que les terpolymères obtenus à partir de trois sortes de monomères.

Les polymères anioniques associatifs utilisés de préférence dans l'invention sont obtenus par copolymérisation d'un monomère (a) choisi parmi les acides carboxyliques à insaturation α-éthylénique (monomère a'), avec un monomère (b) à insaturation éthylénique non tensioactif différent de (a) et/ou un monomère (c) à insaturation éthylénique issu de la réaction d'un monomère acrylique à insaturation α-monoéthylénique ou d'un monomère isocyanate à insaturation monoéthylénique avec un composant amphiphile non ionique monohydrique ou avec une amine grasse primaire ou secondaire.

Comme polymères anioniques comportant au moins une chaîne hydrophobe, pouvant être utilisés dans les compositions de l'invention, on peut citer notamment :
- le terpolymère acide acrylique/acrylate d'éthyle/acrylate d'alkyle, tel que le produit en dispersion aqueuse à 30 % commercialisé sous la dénomination Acusol 823 par la société Rohm & Haas ;
- le copolymère acrylates/steareth-20 methacrylate tel que le produit commercialisé sous la dénomination Aculyn 22 par la société Rohm & Haas ;
- le terpolymère acide (méth)acrylique / acrylate d'éthyle /méthacrylate de béhényle oxyéthyléné (25 OE), tel que le produit en émulsion aqueuse commercialisé sous la dénomination Aculyn 28 par la société Rohm & Haas ;
- le copolymère acide acrylique/itaconate de mono-cétyle oxyéthyléné (20 OE), tel que le produit en dispersion aqueuse à 30 % commercialisé sous la dénomination Structure 3001 par la société National Starch ;
- le copolymère acide acrylique/itaconate de mono-stéaryle oxyéthyléné (20 OE) tel que le produit en dispersion aqueuse à 30 % commercialisé sous la dénomination Structure 2001 par la société National Starch ;
- le copolymère acrylates/acrylate modifié par des alcools en C12-C24 polyoxyéthylénés (25 OE), tel que le latex à 30-32 % de copolymère, commercialisé sous la dénomination Synthalen W2000 par la société 3V SA ;
- le terpolymère acide méthacrylique/acrylate de méthyle/diméthylmétaisopropényl benzylisocyanate d'alcool béhénylique éthoxylé, tel que le produit en dispersion aqueuse à 24 % et comportant 40 groupes oxyéthylénés, décrit dans le document EP-A-0 173 109.

L'addition de neutralisants peut s'avérer utile pour augmenter la solubilité des polymères dans l'eau. On peut alors utiliser tout neutralisant connu, et en particulier on peut le choisir parmi les bases inorganiques telles que la soude, la potasse, l'ammoniac, et parmi les bases organiques telles que la mono-, di- et tri-éthanolamine, l'aminométhylpropanediol-1,3, la N-méthylglucamine, les acides aminés basiques comme l'arginine et la lysine, et leurs mélanges. La quantité de neutralisant dépend du polymère utilisé et des autres constituants de la formule. Elle peut aller par exemple de 0,01 à 5 % et mieux de 0,05 à 5 % du poids total de la composition.

A titre de gélifiants, on peut citer les polymères hydrosolubles et totalement exempts de chaîne hydrophobe choisis par exemple parmi les homopolymères et copolymères d'oxyde d'éthylène ; les alcools polyvinyliques ; les homopolymères et copolymères de vinylpyrrolidone ; les homopolymères et copolymères de vinylcaprolactame ; les homopolymères et copolymères de polyvinylméthyléther ; les homopolymères et copolymères acryliques non-ioniques ; les alkyl-C₁-C₂-celluloses et leurs dérivés, l'hydroxymethylpropylcellulose; les alkyl-C₁-C₃-guar ou hydroxyalkyl-C₁-C₃-guar.

On peut également citer comme gélifiants : les silices nanométriques de type Aerosil, les alcools gras tels que les alcools stéarylique, cétylique, béhénique, les dérivés d'algues tels que le satiagum, des gommes naturelles telles que l'adragante, les argiles, les gommes polysaccharides comme la gome de xanthane et des polymères synthétiques tels que les mélanges d'acides polycarboxyvinyliques commercialisés sous la dénomination CARBOPOL par la société GOODRICH et le mélange de copolymères acrylate de Na/acrylamide commercialisé sous la dénomination HOSTACERIN PN 73 par la société HOECHST.

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Les compositions cosmétiques selon l'invention peuvent par exemple être utilisées comme produit de soin et/ou de protection solaire pour le visage et/ou le corps. de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés.

| **Exemple 1** | |
|---|---|
| *Phase A* | |
| AQ38S (Eastman Chemical) | 2,0% |
| Glycérine | 5,0% |
| Conservateurs | 1,2% |
| Séquestrant | 0,1% |
| Eau déminéralisée | 49.2% |
| | |

| *Phase B* | |
|---|---|
| Huile de vaseline | 10,0 % |
| octyl-methoxycinnamate | 7,0% |
| 1,1-dicarboxy-(2'2'-diméthyl-propyl)-4,4-diphénylbutadiène (composé f) | 5,0 % |
| Octyldodécanol | 5,0% |
| Atky)C₁₂-₁₅benzoate | 15% |

On mélange les constituants de la phase A et on chauffe le mélange à 70°C sous agitation magnétique jusqu'à dispersion complète du polymère, puis on refroidit la solution jusqu'à température ambiante. On prépare par ailleurs la phase B. On introduit la phase A dans la phase B sous vive agitation. On homogénéise l'émulsion sous une pression de 600 bars (2 à 4 passages) en ramenant l'émulsion à la température ambiante entre chaque passage. La taille des gouttes d'huile est de l'ordre de 250 nm.

On obtient une émulsion fluide, stable, sprayable apte à protéger la peau contre les rayons du soleil.

## Revendications

1. Emulsion huile-dans-eau dans laquelle les globules d'huile de l'émulsion ont une taille moyenne d'au plus 500 nm contenant au moins des particules de polymère ionique et au moins un système filtrant les radiations UV, **caractérisée par le fait que** le système filtrant comprend au moins un filtre UV-A du type 4,4-diarylbutadiène.

2. Emulsion selon la revendication 1, **caractérisée en ce qu'**elle est exempte de tensioactif.

3. Emulsion selon la revendication 1 ou 2, **caractérisée en ce que** le polymère ionique est choisi parmi les polymères anioniques ou cationiques ou leurs mélanges, et les mélanges d'au moins un polymère ionique et d'au moins un polymère non-ionique.

4. Emulsion selon l'une quelconque des revendications précédentes, **caractérisée en ce que** le polymère ionique est un polymère anionique.

5. Emulsion selon la revendication 4, **caractérisée en ce que** le polymère ionique est un copolymère d'acide isophtalique et/ou d'acide sulfoisophtalique.

6. Emulsion selon la revendication 5, **caractérisée en ce que** le polymère ionique est un copolymère de Phtalate /Sulfoisophtalate /Glycol

7. Emulsion selon la revendication 6, **caractérisée en ce que** le polymère ionique est un copolymère de Diéthylèneglycol/Phtalate/Isophtalate/1,4-cyclohexane-diméthanol.

8. Emulsion selon l'une quelconque des revendications 5 à 7, où la proportion de motifs dérivés d'acide sulfoisophtalique varie de préférence de 2 à 20 % en poids par rapport au poids total du polymère.

9. Emulsion selon l'une quelconque des revendications 1 à 3, où le polymère ionique hydrodispersible est choisi parmi les copolymères vinyliques filmogènes utilisés couramment pour la préparation de compositions cosmétiques.

10. Emulsion selon la revendication 9, **caractérisée en ce que** les copolymères vinyliques filmogène sont choisis parmi
(i) les copolymères acétate de vinyle/acide crotonique polyéthoxylés ;
(ii) les copolymères acétate de vinyle/acide crotonique ;
(iii) les terpolymères acétate de vinyle/acide crotonique/néodécanoate de vinyle ;
(iv) les copolymères N-octylacrylamide/méthacrylate de méthyle/méthacrylate d'hydroxypropyle/acide acrylique/méthacrylate de tert-butylaminoéthyle ;
(v) les copolymères alternés méthylvinyléther/anhydride maléique monoestérifiés par le butanol ;
(vi) les terpolymères acide acrylique/acrylate d'éthyle/N-tert-butylacrylamide .

11. Emulsion selon l'une quelconque des revendications 1 à 3, où le polymère ionique hydrodispersible est choisi parmi les polymères anioniques hydrodispersibles naturels

12. Emulsion selon la revendication 11, **caractérisée en ce que** les polymères anioniques hydrodispersibles naturels sont choisis parmi la résine shellac, la gomme de sandaraque et les dammars.

13. Emulsion selon l'une quelconque des revendications 1 à 12, où le polymère ionique hydrodispersible a une masse molaire moyenne en poids allant de 1000 à 5 000 000.

14. Emulsion selon l'une quelconque des revendications précédentes, **caractérisée en ce que** les particules de polymère ionique hydrodispersible ont une granulométrie allant de 10 à 400 nanomètres.

15. Emulsion selon l'une quelconque des revendications précédentes, **caractérisée en ce que** les particules de polymère ionique représentent de 0,1 à 10 % du poids total de l'émulsion.

16. Emulsion selon l'une quelconque des revendications précédentes, **caractérisée en ce que** le rapport en poids des particules de polymère ionique à la phase huileuse va de 1/5 à 1/40.

17. Emulsion selon l'une quelconque des revendications précédentes, **caractérisée en ce que** la phase huileuse représente de 0,1 à 45 % du poids total de l'émulsion.

18. Emulsion selon l'une quelconque des revendications 1 à 17, où le filtre UV-A du type 4,4-diarylbutadiène répond à la formule (I) suivante : 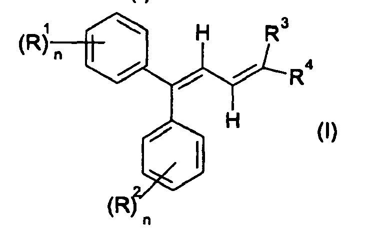 dans laquelle le système diène est de configuration Z,Z ; Z,E ; E,Z ou E,E ou des mélanges desdites configurations et où :
- R¹ et R², identiques ou différents, désignent hydrogène, un radical alkyle en C1-C20,; un radical alcényle en C₂- C₁₀ ; un radical alcoxy en C₁-C₁₂ ; un radical cycloalkyle en C₃-C₁₀ ; un radical cycloalcényle en C₃-C₁₀ ; un radical alcoxycarbonyle en C₁-C₂₀ ; un radical monoalkylamino en C₁-C₁₂ ; un radical dialkylamino en C₁-C₁₂ ; un aryle ; un hétéroaryle ou un substituant hydrosolubilisant choisi parmi un reste carboxylate, sulfonate ou un reste ammonium ;
- R³ désigne un groupe COOR⁵ ; COR⁵ ; CONR⁵R⁶; CN ; O=S(-R⁵)=O ; O=S(-OR⁵)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; un radical bicycloalcényle en C₇-C₁₀ ; un aryle en C₆-C₁₈ éventuellement substitué ; un hétéroaryle en C₃-C₇ éventuellement substitué;
- R⁴ désigne un groupe COOR⁶; COR⁶; CONR⁵R⁶ ; CN ; O=S(-R⁶)=O ; O=S(-OR⁶)=O ; R⁷O-P-(-OR⁸)=O; un radical alkyle en C₁-C₂₀ ; un radical alcényle en C₂- C₁₀ ; un radical cycloalkyle en C₃-C₁₀ ; un radical bicycloalkyle en C₇-C₁₀ ; un radical cycloalcényle en C₃-C₁₀; un radical bicycloalcényle en C₇-C₁₀ ; un aryle en C₆-C₁₈ éventuellement substitué ; un hétéroaryle en C₃-C₇ éventuellement substitué;
- les radicaux R⁵ à R⁸, identiques ou différents, désignent hydrogène ; un radical alkyle en C₁-C₂₀ ; un radical alcényle en C₂-C₁₀ ; un radical cycloalkyle en C₃-C₁₀; un radical bicycloalkyle en C₇-C₁₀ ; un radical bicycloalcényle en C₃-C₁₀ ; un radical cycloalcényle en C₇-C₁₀ ; un aryle éventuellement substitué ; un hétéroaryle éventuellement substitué ;
- n varie de 1 à 3 ;
les radicaux R³ à R⁸ peuvent former entre eux avec les atomes de carbone auxquels ils sont liés, un noyau en C₅-C₆ pouvant être condensé.

19. Emulsion selon la revendication 18, où le composé de formule (I) est chois parmi ceux pour lesquels
- n = 1 ou 2 ;
- R¹ et R², identiques ou différents, désignent hydrogène, un radical alkyle en C₁-C_{20,}; un radical alcoxy en C₁-C₁₂ ; un radical monoalkylamino en C₁-C₁₂ ; un radical dialkylamino en C₁-C₁₂ ; un substituant hydrosolubilisant choisi parmi un groupe carboxylate, un groupe sulfonate ou un reste ammonium ;
- R³ désigne un groupe COOR⁵; COR⁵ ; CONR⁵R⁶; un radical alkyle en C₁-C₂₀ ; un radical cycloalkyle en C₃-C₁₀ ; un radical cycloalcényle en C₃-C₁₀ un radical bicycloalkyle en C₇-C₁₀ ; phényle, naphtyle ou thienyle éventuellement substitué ;
- R⁴ désigne un groupe COOR⁶ ; COR⁶ ; CONR⁵R⁶; un radical alkyle en C₁-C₂₀ ; un radical cycloalkyle en C₃-C₆ ; un radical cycloalcényle en C₃-C₁₀ ; un radical bicycloalkyle en C₇-C₁₀ ; phényle, naphtyle ou thienyle éventuellement substitué ;
- les radicaux R et R⁶, identiques ou différents, désignent hydrogène ; un radical alkyle en C₁-C₁₂ ; un radical cycloalkyle en C₃-C₁₀ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.

20. Emulsion selon la revendication 19, où le composé de formule (I) est chois parmi ceux pour lesquels
- R¹ et R², identiques ou différents, désignent hydrogène, un radical alkyle en C₁-C₂₀ ; un radical alcoxy en C₁-C₂₀ ; un substituant hydrosolubilisant choisi parmi un groupe carboxylate, un groupe sulfonate ou un reste ammonium ;
- R³ désigne un groupe COOR⁵ ; COR⁵ ; CONR⁵R⁶ ;
- R⁴ désigne un groupe COOR⁶ ; COR⁶ ; CONR⁵R⁶ ;
- les radicaux R et R⁶, identiques ou différents, désignent hydrogène ; un radical alkyle en C₁-C₁₂ ; un radical cycloalkyle en C₃-C₆ ; un radical cycloalcényle en C₃-C₁₀ ; un radical bicycloalkyle en C₇-C₁₀ ; un radical bicycloalcényle en C₃-C₁₀ ; phényle ou naphtyle éventuellement substitué.

21. Emulsion selon la revendication 20, où le composé de formule (I) est choisi parmi ceux de formule (I') suivante : 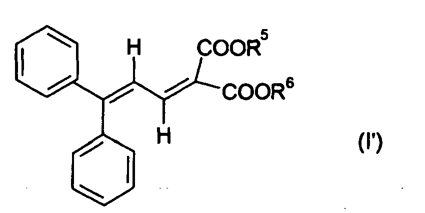 où les radicaux R⁵ et R⁶, identiques ou différents, désignent hydrogène ; un radical alkyle en C₁-C₂₀ ; un radical cycloalkyle en C₃-C₆ ; un radical cycloalcényle en C₃-C₁₀.

22. Emulsion selon la revendication 21, où le composé de formule (I') est le 1,1-dicarboxy-(2'2'-diméthyl-propyl)-4,4-diphénylbutadiène de structure :

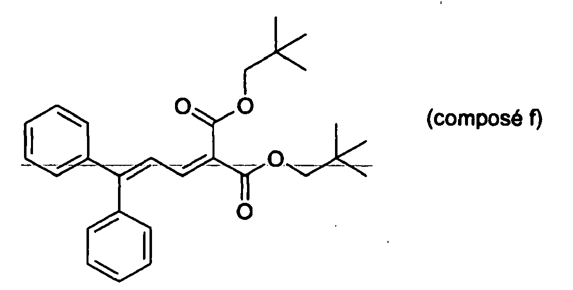

23. Emulsion selon l'une quelconque des revendications 1 à 17, où le filtre UV-A du type 4,4-diarylbutadiène répond à la formule (II) suivante : 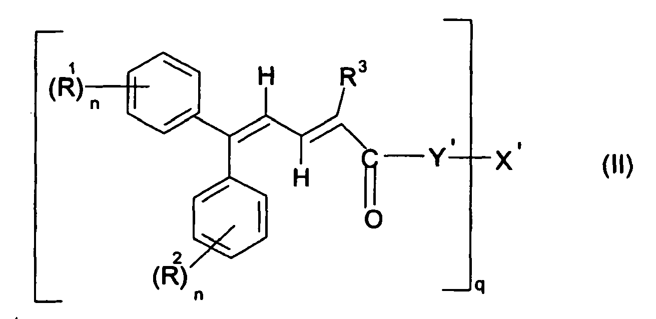 dans laquelle le système diène est de configuration Z,Z ; Z,E ; E,Z ou E,E ou des mélanges desdites configurations et où :
- R¹, R², R³ et n ont les mêmes significations indiquées dans la formule (I) telle que définie dans la revendication 18;
- Y' désigne un groupe -O- ou -NR⁹-
- R⁹ désigne hydrogène ; un radical alkyle en C₁-C₂₀, linéaire ou ramifié ; un radical alcényle en C₂- C₁₀ ; un radical cycloalkyle en C₃-C₁₀ ; un radical bicycloalkyle en C₇-C₁₀ ; un radical cycloalcényle en C₃-C₁₀ ; ; un radical bicycloalcényle en C₇-C₁₀ ; un aryle ; un hétéroaryle;
- X' désigne un reste de polyol en C₂-C₂₀ linéaire ou ramifié, aliphatique ou cycloaliphatique comprenant de 2 à10 groupes hydroxy et de valence q ; la chaîne carbonée dudit reste pouvant être interrompue par un ou plusieurs atomes de souffre ou d'oxygène ; un ou plusieurs groupes imines ; un ou plusieurs alkylimino en C₁-C₄ ;
- q varie de 2 à 10.

24. Emulsion selon la revendication 23, où le composé de formule (II) est choisi parmi ceux pour lesquels :
- R¹ et R², identiques ou différents, désignent hydrogène, un radical alkyle en C₁-C₁₂ ; un radical alcoxy en C₁-C₈ ; un substituant hydrosolubilisant choisi parmi un groupe carboxylate, un groupe sulfonate ou un reste ammonium ;
- R³ désigne un groupe COOR⁵ ; CONR⁵R⁶ ; CN ; un radical cycloalkyle en C₃-C₁₀ ; un radical bicycloalkyle en C₇-C₁₀ ;
- R⁵ et R⁶ , identiques ou différents, désignent un radical alkyle en C₁-C_{20.} linéaire ou ramifié ; un radical cycloalkyle en C₃-C₁₀ ; un radical bicycloalkyle en C₇-C₁₀; naphtyle ou phényle éventuellement substitué ;
- X' désigne un reste de polyol en C₂-C₂₀ comprenant de 2 à 6 groupes hydroxy et plus particulièrement de 2 à 4.

25. Emulsion selon la revendication 24, où le composé de formule (II) est choisi parmi ceux pour lesquels X' désigne un reste de pentaerythritol.

26. Emulsion selon la revendication 25, où le composé de formule (II) est choisi parmi les composés suivants :

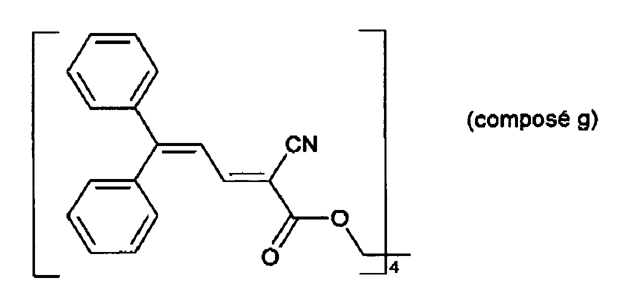

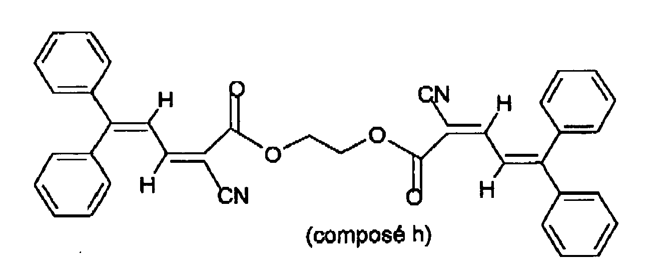

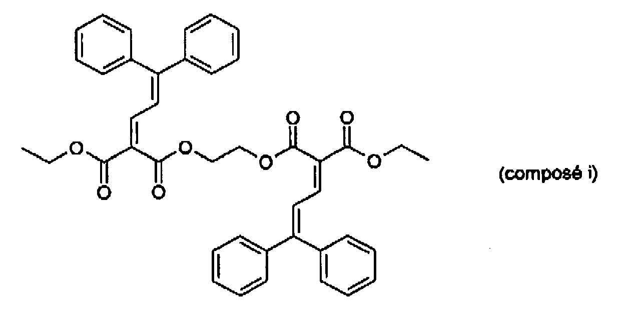

27. Emulsion selon l'une quelconque des revendications 1 à 26, **caractérisée en ce que** le ou les composés 4,4-diarylbutadiène sont présents dans des proportions allant de 0,1 % à 20% en poids, plus préférentiellement de 1 à 10% en poids par rapport au poids total de l'émulsion.

28. Emulsion selon l'une quelconque des revendications 1 à 27, **caractérisée en ce qu'**elle contient en outre au moins un filtre solaire organique ou inorganique complémentaire actif dans l'UV-A et/ou l'UV-B, hydrosolubles, liposolubles ou bien insolubles dans les solvants cosmétiques couramment utilisés.

29. Emulsion selon la revendication 28, ou les filtres organiques complémentaires sont choisis parmi les anthranilates ; les dérivés cinnamiques; les dérivés de dibenzoylméthane ; les dérivés salicyliques, les dérivés du camphre ; les dérivés de triazine ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de benzoxazole ; les dérivés de méthylène bis-(hydroxyphényl benzotriazole) ; les polymères filtres et silicones filtres ; les dimères dérivés d'α-alkylstyrène et leurs mélanges.

30. Emulsion selon la revendication 29, ou les filtres organiques complémentaires sont choisis parmi
Ethylhexyl Salicylate,
Ethylhexyl Methoxycinnamate Octocrylene,
Butyl Methoxydibenzoylmethane Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle 4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
2,4-bis-[5-1(diméthylpropyl)benzoxazol-2-yl-(4-phenyl)-imino]-6-(2-ethylhexyl)-imino-1,3,5-triazine et leurs mélanges.

31. Emulsion selon la revendication 28, où les filtres complémentaires inorganiques sont choisis parmi des pigments ou des nanopigments d'oxydes métalliques, enrobés ou non.

32. Emulsion selon la revendication 31, où les filtres complémentaires inorganiques sont des nanopigments d'oxyde de titane, amorphe ou cristallisé, sous forme rutile et/ou anatase, de fer, de zinc, de zirconium ou de cérium

33. Emulsion selon l'une quelconque des revendications 1 à 32, **caractérisée en ce qu'**elle contient en outre au moins un agent de bronzage et/ou de brunissage artificiel de la peau.

34. Emulsion selon l'une quelconque des revendications 1 à 33, **caractérisée en ce qu'**elle contient en outre au moins un adjuvant cosmétique choisi parmi les solvants organiques, les épaississants et/ou gélifiants ioniques ou non ioniques, les adoucissants, les humectants, les opacifiants, les stabilisants, les émollients, les silicones, les agents répulsifs contre les insectes, les parfums, les conservateurs, les tensioactifs, les chargeas, les pigments, les polymères, les propulseurs, les agents alcalinisants ou acidifiants ou tout autre ingrédient habituellement utilisé dans le domaine cosmétique et/ou dermatologique.

35. Emulsion selon l'une quelconque des revendications 1 à 34, **caractérisée en ce qu'**elle comporte au plus 1% en poids par rapport au poids total de la composition en tensioactif émulsionnant et voire exempte de tensioactif émulsionnant.

36. Emulsion selon l'une quelconque des revendications 1 à 34, **caractérisée en ce qu'**elle comporte en plus au moins un agent gélifiant.

37. Emulsion selon la revendication 36, où le ou les agents gélifiants sont utilisés à des concentrations allant de 0,1 à 10 %, de préférence 0,1 à 5 % et mieux de 0,1 à 3 % du poids total de l'émulsion.

38. Emulsion selon la revendication 36 ou 37, où l'agent gélifiant est choisi parmi les polymères associatifs non-ioniques ou anioniques hydrosolubles ou hydrodispersibles comportant au moins une séquence hydrophobe et au moins une séquence hydrophile.

39. Emulsion selon la revendication 36 ou 37, où les polymères associatifs non-ioniques sont choisis parmi :
**(1)** Les celluloses modifiées par des groupements comportant au moins une chaîne hydrophobe ;
**(2)** Les hydroxypropylguars modifiés par des groupements comportant au moins une chaîne grasse en C₁₀-C₃₀ ;
**(3)** Les uréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques ;
**(4)** Les copolymères de vinyl pyrrolidone et de monomères hydrophobes à chaîne grasse
**(5)** Les copolymères de méthacrylates ou d'acrylates d'alkyles en C₁-C₆ et de monomères amphiphiles comportant au moins une chaîne grasse ;
**(6)** les copolymères de méthacrylates ou d'acrylates hydrophiles et de monomères hydrophobes comportant au moins une chaîne grasse.

40. Emulsion selon la revendication 38, où les polymères associatifs anioniques sont choisis parmi les copolymères d'acide acrylique ou méthacrylique comportant une séquence hydrophile et au moins une séquence hydrophobe.

41. Emulsion selon la revendication 38, où les polymères associatifs anioniques sont choisis parmi
- le terpolymère acide acrylique/acrylate d'éthyle/acrylate d'alkyle ;
- le copolymère acrylates/steareth-20 methacrylate ;
- le terpolymère acide (méth)acrylique / acrylate d'éthyle /méthacrylate de béhényle oxyéthyléné (25 OE) ;
- le copolymère acide acrylique/itaconate de mono-cétyle oxyéthyléné (20 OE) ;
- le copolymère acide acrylique/itaconate de mono-stéaryle oxyéthyléné (20 OE) ;
- le copolymère acrylates/acrylate modifié par des alcools en C₁₂-C₂₄ polyoxyéthylénés (25 OE) ;
- le terpolymère acide méthacrylique/acrylate de méthyle/diméthylmétaisopropényl benzylisocyanate d'alcool béhénylique éthoxylé.

42. Emulsion selon la revendication 36 ou 37, où l'agent gélifiant est choisi parmi les polymères hydrosolubles totalement exempts de chaîne hydrophobe.

43. Emulsion selon la revendication 42, où les polymères hydrosolubles et totalement exempts de chaîne hydrophobesont choisis parmi les homopolymères et copolymères d'oxyde d'éthylène ; les alcools polyvinyliques ; les homopolymères et copolymères de vinylpyrrolidone ; les homopolymères et copolymères de vinylcaprolactame ; les homopolymères et copolymères de polyvinylméthyléther ; les homopolymères et copolymères acryliques non-ioniques ; les alkyl-C₁-C₂-celluloses et leurs dérivés, l'hydroxymethylpropylcellulose; les alkyl-C₁-C₃-guar ou hydroxyalkyl-C₁-C₃-guar.

44. Emulsion selon la revendication 36 ou 37, où l'agent gélifiant est choisi parmi les silices nanométriques , les alcools gras tels , les dérivés d'algues, des gommes naturelles telles que l'adragante, les argiles, les gommes polysaccharides et des polymères synthétiques.

45. Procédé de fabrication d'une émulsion telle que définie dans les revendications 1 à **44,** consistant dans une première étape à mélanger sous agitation la phase aqueuse, la phase huileuse et les particules de polymère et, dans une seconde étape à soumettre le mélange obtenu à une homogénéisation basée sur le principe de la cavitation.

46. Procédé de fabrication selon la revendication 45, **caractérisé en ce que** dans la seconde étape, l'homogénéisation est réalisée sous une pression allant de 400 à 700 bars.

47. Utilisation d'une composition telle que définie selon l'une quelconque des revendications 1 à 43 pour la fabrication de produits pour le traitement cosmétique de la peau, des lèvres et des cheveux, y compris le cuir chevelu, en particulier pour la protection et/ou le soin de la peau, des lèvres et/ou des cheveux et/ou pour le maquillage de la peau et/ou des lèvres.

## Claims

1. Oil-in-water emulsion in which the oil globules of the emulsion have an average size of at most 500 nm, containing at least particles of ionic polymer and at least one UV radiation-screening system, **characterized in that** the screening system comprises at least one UV-A-screening agent of the 4,4-diarylbutadiene type.

2. Emulsion according to Claim 1, **characterized in that** it is free of surfactant.

3. Emulsion according to Claim 1 or 2, **characterized in that** the ionic polymer is chosen from anionic or cationic polymers or mixtures thereof, and mixtures of at least one ionic polymer and at least one nonionic polymer.

4. Emulsion according to any one of the preceding claims, **characterized in that** the ionic polymer is an anionic polymer.

5. Emulsion according to Claim 4, **characterized in that** the ionic polymer is a copolymer of isophthalic acid and/or sulphoisophthalic acid.

6. Emulsion according to Claim 5, **characterized in that** the ionic polymer is a copolymer of phthalate/sulphoisophthalate/glycol.

7. Emulsion according to Claim 6, **characterized in that** the ionic polymer is a copolymer of diethylene glycol/phthalate/isophthalate/1,4-cyclohexanedimethanol.

8. Emulsion according to any one of Claims 5 to 7, where the proportion of units derived from sulphoisophthalic acid preferably varies from 2 to 20% by weight relative to the total weight of the polymer.

9. Emulsion according to any one of Claims 1 to 3,
where the water-dispersible ionic polymer is chosen from film-forming vinyl copolymers commonly used for the preparation of cosmetic compositions.

10. Emulsion according to Claim 9, **characterized in that** the film-forming vinyl copolymers are chosen from:
(i) polyethoxylated vinyl acetate/crotonic acid copolymers;
(ii) vinyl acetate/crotonic acid copolymers;
(iii) vinyl acetate/crotonic acid/vinyl neodecanoate terpolymers;
(iv) N-octylacrylamide/methyl methacrylate/hydroxy-propyl methacrylate/acrylic acid/tert-butylaminoethyl methacrylate copolymers;
(v) methyl vinyl ether/maleic anhydride alternating copolymers monoesterified with butanol;
(vi) acrylic acid/ethyl acrylate/N-tert-butylacrylamide terpolymers.

11. Emulsion according to any one of Claims 1 to 3, where the water-dispersible ionic polymer is chosen from natural water-dispersible anionic polymers.

12. Emulsion according to Claim 11, **characterized in that** the natural water-dispersible anionic polymers are chosen from shellac resin, sandarac gum and dammars.

13. Emulsion according to any one of Claims 1 to 12, where the water-dispersible ionic polymer has a weight-average molar mass ranging from 1 000 to 5 000 000.

14. Emulsion according to any one of the preceding claims, **characterized in that** the particles of water-dispersible ionic polymer have a particle size ranging from 10 to 400 nanometres.

15. Emulsion according to any one of the preceding claims, **characterized in that** the particles of ionic polymer represent from 0.1 to 10% of the total weight of the emulsion.

16. Emulsion according to any one of the preceding claims, **characterized in that** the weight ratio of the particles of ionic polymer to the oily phase ranges from 1/5 to 1/40.

17. Emulsion according to any one of the preceding claims, **characterized in that** the oily phase represents from 0.1 to 45% of the total weight of the emulsion.

18. Emulsion according to any one of Claims 1 to 17, where the UV-A-screening agent of the 4,4-diarylbutadiene type corresponds to the following formula (I): 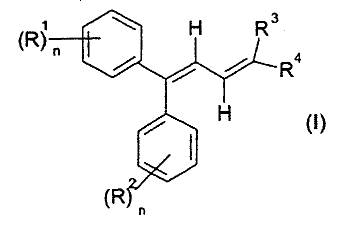 in which the diene system is of the Z,Z; Z,E; E,Z or E,E configuration or mixtures of the said configurations, and where:
- R¹ and R², which are identical or different, denote hydrogen, a C₁-C₂₀ alkyl radical; a C₂-C₁₀ alkenyl radical; a C₁-C₁₂ alkoxy radical; a C₃-C₁₀ cycloalkyl radical; a C₃-C₁₀ cycloalkenyl radical; a C₁-C₂₀ alkoxycarbonyl radical; a C₁-C₁₂ monoalkylamino radical; a C₁-C₁₂ dialkylamino radical; an aryl; a heteroaryl or a water-solubilizing substituent chosen from a carboxylate residue, a sulphonate residue or an ammonium residue;
- R³ denotes a group COOR⁵; COR⁵; CONR⁵R⁶; CN; O=S(-R⁵)=O; O=S(-OR⁵)=O; R⁷O-P-(-OR⁸)=O; a C₁-C₂₀ alkyl radical; a C₂-C₂₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an optionally substituted C₆-C₁₈ aryl; an optionally substituted C₃-C₇ heteroaryl;
- R⁴ denotes a group COOR⁶; COR⁶; CONR⁵R⁶; CN; O=S(-R⁶)=O; O=S(-OR⁶)=O; R⁷O-P-(-OR⁸)=O; a C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an optionally substituted C₆-C₁₈ aryl; an optionally substituted C₃-C₇ heteroaryl;
- the radicals R⁵ to R⁸, which are identical or different, denote hydrogen; a C₁-C₂₀ alkyl radical; a C₂-C₂₀ alkenyl radical; a C₃-C₁₀ cycloalkyl radical; a C₇-C₁₀ bicycloalkyl radical; a C₃-C₁₀ bicycloalkenyl radical; a C₇-C₁₀ cycloalkenyl radical; an optionally substituted aryl; an optionally substituted heteroaryl;
- n varies from 1 to 3;
the radicals R³ to R⁸ can form between them, with the carbon atoms to which they are attached, a C₅-C₆ ring which may be fused.

19. Emulsion according to Claim 18, where the compound of formula (I) is chosen from those for which
- n = 1 or 2;
- R¹ and R², which are identical or different, denote hydrogen, a C₁-C₂₀ alkyl radical; a C₁-C₁₂ alkoxy radical; a C₁-C₁₂ monoalkylamino radical; a C₁-C₁₂ dialkylamino radical; a water-solubilizing substituent chosen from a carboxylate group, a sulphonate group or an ammonium residue;
- R³ denotes a group COOR⁵; COR⁵; CONR⁵R⁶; a C₁-C₂₀ alkyl radical; a C₃-C₁₀ cycloalkyl radical; a C₃-C₁₀ cycloalkenyl radical; a C₇-C₁₀ bicycloalkyl radical; optionally substituted phenyl, naphthyl or thienyl;
- R⁴ denotes a group COOR⁶; COR⁶; CONR⁵R⁶; a C₁-C₂₀ alkyl radical; a C₃-C₆ cycloalkyl radical; a C₃-C₁₀ cycloalkenyl radical; a C₇-C₁₀ bicycloalkyl radical; optionally substituted phenyl, naphthyl or thienyl;
- the radicals R⁵ and R⁶, which are identical or different, denote hydrogen; a C₁-C₁₂ alkyl radical; a C₃-C₁₀ cycloalkyl radical; a C₃-C₁₀ cycloalkenyl radical; a C₇-C₁₀ bicycloalkyl radical; a C₃-C₁₀ bicycloalkenyl radical; optionally substituted phenyl or naphthyl.

20. Emulsion according to Claim 19, where the compound of formula (I) is chosen from those for which
- R¹ and R², which are identical or different, denote hydrogen, a C₁-C₂₀ alkyl radical; a C₁-C₂₀ alkoxy radical; a water-solubilizing substituent chosen from a carboxylate group, a sulphonate group or an ammonium residue;
- R³ denotes a group COOR⁵; COR⁵; CONR⁵R⁶;
- R⁴ denotes a group COOR⁶; COR⁶; CONR⁵R⁶_{;}
- the radicals R⁵ and R⁶, which are identical or different, denote hydrogen; a C₁-C₁₂ alkyl radical; a C₃-C₆ cycloalkyl radical; a C₃-C₁₀ cycloalkenyl radical; a C₇-C₁₀ bicycloalkyl radical; a C₃-C₁₀ bicycloalkenyl radical; optionally substituted phenyl or naphthyl.

21. Emulsion according to Claim 20, where the compound of formula (I) is chosen from those of the following formula (I'): 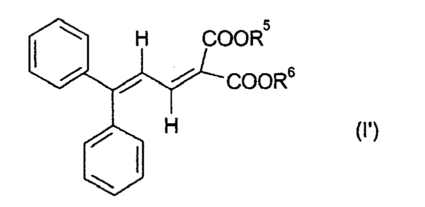 where the radicals R⁵ and R⁶, which are identical or different, denote hydrogen; a C₁-C₂₀ alkyl radical; a C₃-C₆ cycloalkyl radical; a C₃-C₁₀ cycloalkenyl radical.

22. Emulsion according to Claim 21, where the compound of formula (I') is 1,1-dicarboxy(2',2'-dimethylpropyl)-4,4-diphenylbutadiene having the structure:

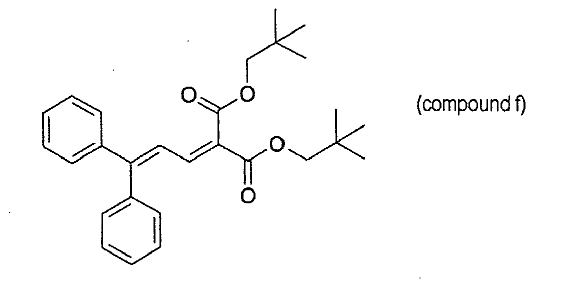

23. Emulsion according to any one of Claims 1 to 17, where the UV-A-screening agent of the 4,4-diarylbutadiene type corresponds to the following formula (II): 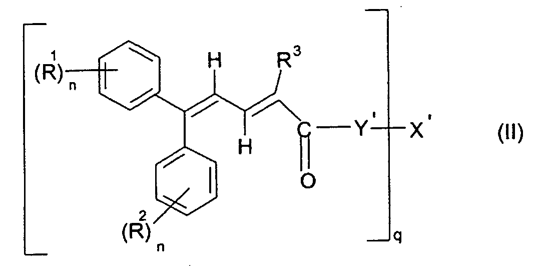 in which the diene system is of the Z,Z; Z,E; E,Z or E,E configuration or mixtures of the said configurations and where:
- R¹, R², R³ and n have the meanings indicated in the formula (I) as defined in Claim 18;
- Y' denotes a group -O- or -NR⁹-
- R⁹ denotes hydrogen; a linear or branched C₁-C₂₀ alkyl radical; a C₂-C₁₀ alkenyl radical; a C₃-C₁₀ cycloalkyl radical; a C₇-C₁₀ bicycloalkyl radical; a C₃-C₁₀ cycloalkenyl radical; a C₇-C₁₀ bicycloalkenyl radical; an aryl; a heteroaryl;
- X' denotes a residue of a linear or branched, aliphatic or cycloaliphatic C₂-C₂₀ polyol comprising from 2 to 10 hydroxyl groups and having the valency q; it being possible for the carbon chain of the said residue to be interrupted by one or more sulphur or oxygen atoms; one or more imine groups; one or more C₁-C₄ alkylimino groups;
- q ranges from 2 to 10.

24. Emulsion according to Claim 23, where the compound of formula (II) is chosen from those for which:
- R¹ and R², which are identical or different, denote hydrogen, a C₁-C₁₂ alkyl radical; a C₁-C₈ alkoxy radical; a water-solubilizing substituent chosen from a carboxylate group, a sulphonate group or an ammonium residue;
- R³ denotes a group COOR⁵; CONR⁵R⁶; CN; a C₃-C₁₀ cycloalkyl radical; a C₇-C₁₀ bicycloalkyl radical;
- R⁵ and R⁶, which are identical or different, denote a linear or branched C₁-C₂₀ alkyl radical; a C₃-C₁₀ cycloalkyl radical; a C₇-C₁₀ bicycloalkyl radical; optionally substituted naphthyl or phenyl;
- X' denotes a C₂-C₂₀ polyol residue comprising from 2 to 6 hydroxyl groups and more particularly from 2 to 4.

25. Emulsion according to Claim 24, where the compound of formula (II) is chosen from those for which X' denotes a pentaerythritol residue.

26. Emulsion according to Claim 25, where the compound of formula (II) is chosen from the following compounds:

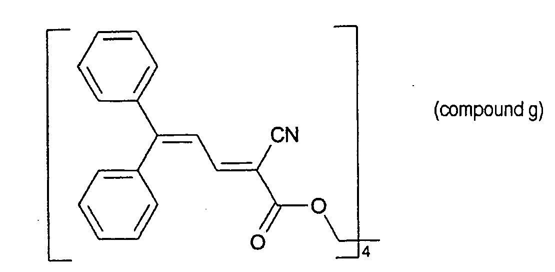

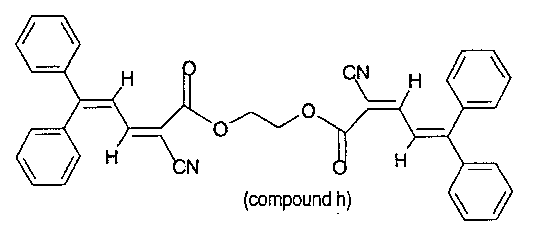

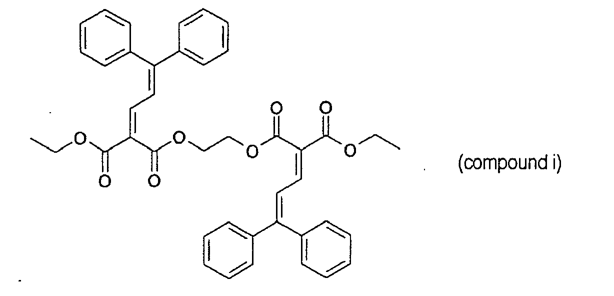

27. Emulsion according to any one of Claims 1 to 26, **characterized in that** the 4,4-diarylbutadiene compound(s) are present in proportions ranging from 0.1% to 20% by weight, more preferably from 1 to 10% by weight relative to the total weight of the emulsion.

28. Emulsion according to any one of Claims 1 to 27, **characterized in that** it furthermore contains at least one additional organic or inorganic sunscreening agent active in UV-A and/or UV-B, water-soluble, fat-soluble or insoluble in the commonly used cosmetic solvents.

29. Emulsion according to Claim 28, where the additional organic screening agents are chosen from anthranilates; cinnamic derivatives; dibenzoylmethane derivatives; salicylic derivatives, camphor derivatives; triazine derivatives; benzophenone derivatives; β,β-diphenyl acrylate derivatives; benzotriazole derivatives; benzalmalonate derivatives; benzimidazole derivatives; imidazolines; bis-benzoazolyl derivatives; p-aminobenzoic acid (PABA) derivatives; benzoxazole derivatives; methylenebis-(hydroxyphenylbenzotriazole) derivatives; screening polymers and screening silicones; dimers derived from α-alkylstyrene and mixtures thereof.

30. Emulsion according to Claim 29, where the additional organic screening agents are chosen from
Ethylhexyl Salicylate,
Ethylhexyl Methoxycinnamate,
Octocrylene,
Butyl Methoxydibenzoylmethane,
Phenylbenzimidazole Sulphonic Acid,
Benzophenone-3,
Benzophenone-4,
Benzophenone-5,
n-Hexyl 2-(4-diethylamino-2-hydroxybenzoyl)benzoate,
4-Methylbenzylidene camphor,
Terephthalylidene Dicamphor Sulphonic Acid,
Disodium Phenyl Dibenzimidazole Tetra-sulphonate,
2,4,6-Tris(4'-diisobutyl aminobenzalmalonate)-s-triazine
Anisotriazine,
Ethylhexyl triazone,
Diethylhexyl Butamido Triazone,
Methylene bis-Benzotriazolyl Tetramethylbutylphenol,
Drometrizole Trisiloxane,
Polysilicone-15,
2,4-Bis-[5-1-(dimethylpropyl)benzoxazol-2-yl-(4-phenyl)imino]-6-(2-ethylhexyl)imino-1,3,5-triazine
and mixtures thereof.

31. Emulsion according to Claim 28, where the additional inorganic screening agents are chosen from pigments or nanopigments of metal oxides, coated or uncoated.

32. Emulsion according to Claim 31, where the additional inorganic screening agents are nanopigments of titanium oxide, amorphous or crystallized, in rutile and/or anatase form, iron oxide, zinc oxide, zirconium oxide or cerium oxide.

33. Emulsion according to any one of Claims 1 to 32, **characterized in that** it additionally contains at least one agent for artificial bronzing and/or tanning of the skin.

34. Emulsion according to any one of Claims 1 to 33, **characterized in that** it additionally contains at least one cosmetic adjuvant chosen from organic solvents, ionic or nonionic thickeners and/or gelling agents, demulcents, humectants, opacifying agents, stabilizers, emollients, silicones, insect repellents, perfumes, preservatives, surfactants, fillers, pigments, polymers, propellants, alkanizing or acidifying agents or any other ingredient customarily used in the cosmetic and/or dermatological field.

35. Emulsion according to any one of Claims 1 to 34, **characterized in that** it comprises at most 1% by weight relative to the total weight of the composition as emulsifying surfactant and is even free of emulsifying surfactant.

36. Emulsion according to any one of Claims 1 to 34, **characterized in that** it additionally contains at least one gelling agent.

37. Emulsion according to Claim 36, where the gelling agent(s) are used at concentrations ranging from 0.1 to 10%, preferably 0.1 to 5% and better still from 0.1 to 3% of the total weight of the emulsion.

38. Emulsion according to Claim 36 or 37, where the gelling agent is chosen from water-soluble or water-dispersible nonionic or anionic associative polymers containing at least one hydrophobic sequence and at least one hydrophilic sequence.

39. Emulsion according to Claim 36 or 37, where the nonionic associative polymers are chosen from:
**(1)** celluloses modified with groups containing at least one hydrophobic chain;
**(2)** hydroxypropylguars modified with groups containing at least one C₁₀-C₃₀ fatty chain;
**(3)** polyether urethanes containing, in their chain, both hydrophilic sequences most often of a polyoxyethylenated nature and hydrophobic sequences which may be aliphatic linkages alone and/or cycloaliphatic and/or aromatic linkages;
**(4)** copolymers of vinylpyrrolidone and hydrophobic monomers with a fatty chain;
**(5)** copolymers of C₁-C₆ alkyl methacrylates or acrylates and amphiphilic monomers containing at least one fatty chain;
**(6)** copolymers of hydrophilic methacrylates or acrylates and hydrophobic monomers containing at least one fatty chain.

40. Emulsion according to Claim 38, where the anionic associative polymers are chosen from copolymers of acrylic or methacrylic acid containing a hydrophilic sequence and at least one hydrophobic sequence.

41. Emulsion according to Claim 38, where the anionic associative polymers are chosen from:
- acrylic acid/ethyl acrylate/alkyl acrylate terpolymer;
- acrylates/steareth-20 methacrylate copolymer;
- oxyethylenated (25 EO) (meth)acrylic acid/ethyl acrylate/behenyl methacrylate terpolymer;
- oxyethylenated (20 EO) acrylic acid/monocetyl itaconate copolymer;
- oxyethylenated (20 EO) acrylic acid/monostearyl itaconate copolymer;
- acrylates/acrylate copolymer modified with polyoxyethylenated (25 EO) C₁₂-C₂₄ alcohols;
- ethoxylated methacrylic acid/methyl acrylate/dimethyl-meta-isopropenyl benzyl isocyanate of behenyl alcohol terpolymer.

42. Emulsion according to Claim 36 or 37, where the gelling agent is chosen from water-soluble polymers completely free of a hydrophobic chain.

43. Emulsion according to Claim 42, where the polymers which are water-soluble and completely free of hydrophobic chain are chosen from homopolymers and copolymers of ethylene oxide; polyvinyl alcohols; homopolymers and copolymers of vinylpyrrolidone; homopolymers and copolymers of vinylcaprolactam; homopolymers and copolymers of polyvinyl methyl ether; nonionic acrylic homopolymers and copolymers; C₁-C₂ alkyl celluloses and their derivatives, hydroxymethylpropylcellulose; C₁-C₃ alkylguar or C₁-C₃ hydroxyalkyl-guar.

44. Emulsion according to Claim 36 or 37, where the gelling agent is chosen from nanometric silicas, fatty alcohols such as algal derivatives, natural gums such as tragacanth, clays, polysaccharide gums and synthetic polymers.

45. Method of manufacturing an emulsion as defined in Claims 1 to 44, consisting, in a first step, in mixing, with stirring, the aqueous phase, the oily phase and the polymer particles and, in a second step, in subjecting the mixture obtained to homogenization based on the principle of cavitation.

46. Method of manufacture according to Claim 45, **characterized in that** in the second step, the homogenization is carried out at a pressure ranging from 400 to 700 bar.

47. Use of an emulsion as defined in any one of Claims 1 to 44, for the manufacture of products for the cosmetic treatment of the skin, the lips and the hair, including the scalp, in particular for the protection and/or care of the skin, the lips and/or the hair, and/or for making up the skin and/or the lips.

## Patentansprüche

1. Öl-in-Wasser-Emulsion, bei der die Öltröpfchen der Emulsion eine mittlere Größe von höchstens 500 nm aufweisen und die zumindest Partikel eines ionischen Polymers und zumindest ein Filtersystem für UV-Strahlung enthält, **dadurch gekennzeichnet, dass** das Filtersystem mindestens einen organischen UV-A-Filter vom 4,4-Diarylbutadientyp enthält.

2. Emulsion nach Anspruch 1, **dadurch gekennzeichnet, dass** sie keinen grenzflächenaktiven Stoff enthält.

3. Emulsion nach Anspruch 1 oder 2, **dadurch gekennzeichnet, dass** das ionische Polymer unter den anionischen oder kationischen Polymeren oder deren Gemischen und den Gemischen mindestens eines ionischen Polymers und mindestens eines nichtionischen Polymers ausgewählt ist.

4. Emulsion nach einem der vorhergehenden Ansprüche, **dadurch gekennzeichnet, dass** das ionische Polymer ein anionisches Polymer ist.

5. Emulsion nach Anspruch 4, **dadurch gekennzeichnet, dass** das ionische Polymer ein Copolymer von Isophthalsäure und/oder Sulfoisophthalsäure ist.

6. Emulsion nach Anspruch 5, **dadurch gekennzeichnet, dass** das ionische Polymer ein Copolymer Phthalat/Sulfoisophthalat/Glycol ist.

7. Emulsion nach Anspruch 6, **dadurch gekennzeichnet, dass** das ionische Polymer ein Copolymer Diethylenglycol/Phthalat/Isophthalat/ 1,4-Cyclohexan-dimethanol ist.

8. Emulsion nach einem der Ansprüche 5 bis 7, wobei der Anteil der von Sulfoisophthalsäure abgeleiteten Einheiten vorzugsweise im Bereich von 2 bis 20 Gew.-%, bezogen auf das Gesamtgewicht des Polymers, liegt.

9. Emulsion nach einem der Ansprüche 1 bis 3, wobei das in Wasser dispergierbare ionische Polymer unter den filmbildenden Vinylcopolymeren ausgewählt ist, die häufig für die Herstellung von kosmetischen Zusammensetzungen verwendet werden.

10. Emulsion nach Anspruch 9, **dadurch gekennzeichnet, dass** die filmbildenden Vinylcopolymere ausgewählt sind unter:
(i) polyethoxylierten Copolymeren Vinylacetat/Crotonsäure;
(ii) Copolymeren Vinylacetat/Crotonsäure;
(iii) Terpolymeren Vinylacetat/Crotonsäure/Vinylneodecanoat;
(iv) Copolymeren N-Octylacrylamid/Methylmethacrylat/Hydroxypropylmethacrylat/Acrylsäure/*tert*-Butylaminoethylmethacrylat;
(v) alternierenden, mit Butanol monoveresterten Copolymeren Methylvinylether/Maleinsäureanhydrid;
(vi) Terpolymeren Acrylsäure/Ethylacrylat/N-*tert*-Butylacrylamid.

11. Emulsion nach einem der Ansprüche 1 bis 3, wobei das in Wasser dispergierbare ionische Polymer unter den in Wasser dispergierbaren natürlichen anionischen Polymeren ausgewählt ist.

12. Emulsion nach Anspruch 11, **dadurch gekennzeichnet, dass** die in Wasser dispergierbaren natürlichen anionischen Polymere unter Schellack, Sandarak und Dammarharzen ausgewählt sind.

13. Emulsion nach einem der Ansprüche 1 bis 12, wobei das in Wasser dispergierbare ionische Polymer eine gewichtsmittlere Molmasse von 1000 bis 5 000 000 besitzt.

14. Emulsion nach einem der vorhergehenden Ansprüche, **dadurch gekennzeichnet, dass** die Partikel des in Wasser dispergierbaren ionischen Polymers eine Korngröße von 10 bis 400 nm aufweisen.

15. Emulsion nach einem der vorhergehenden Ansprüche, **dadurch gekennzeichnet, dass** die Partikel des ionischen Polymers 0, 1 bis 10 % des Gesamtgewichts der Emulsion ausmachen.

16. Emulsion nach einem der vorhergehenden Ansprüche, **dadurch gekennzeichnet, dass** das Gewichtsverhältnis der Partikel des ionischen Polymers und der Ölphase im Bereich von 1/5 bis 1/40 liegt.

17. Emulsion nach einem der vorhergehenden Ansprüche, **dadurch gekennzeichnet, dass** die Ölphase 0,1 bis 45 % des Gesamtgewichts der Emulsion ausmacht.

18. Emulsion nach einem der Ansprüche 1 bis 17, wobei der UV-A-Filter vom 4,4-Diarylbutadientyp der folgenden Formel (I) entspricht: 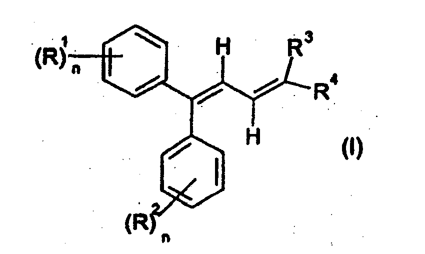 in der das Dien-System die Konfiguration Z,Z; Z,E; E,Z oder E,E aufweist oder Mischungen dieser Konfigurationen bedeutet und in der bedeuten:
- R¹ und R², die gleich oder verschieden sind, Wasserstoff, eine C₁₋₂₀-Alkylgruppe; eine C₂₋₁₀-Alkenylgruppe; eine C₁₋₁₂-Alkoxygruppe; eine C₃₋₁₀-Cycloalkylgruppe; eine C₃₋₁₀-Cycloalkenylgruppe; eine C₁₋₂₀-Alkoxycarbonylgruppe; eine C₁₋₁₂-Monoalkylaminogruppe; eine C₁₋₁₂-Dialkylaminogruppe; Aryl; Heteroaryl oder einen Wasserlöslichkeit vermittelnden Substituenten, der unter Carboxylat, Sulfonat oder Ammonium ausgewählt ist;
- R³ eine Gruppe COOR⁵; COR⁵; CONR⁵R⁶; CN; O=S(-R⁵)=O; O=S(-OR⁵)=O; R⁷O-P-(-OR⁸)=O; eine C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine C₆₋₁₈-Arylgruppe, die gegebenenfalls substituiert ist; eine C₃₋₇-Heteroarylgruppe, die gegebenenfalls substituiert ist;
- R⁴ eine Gruppe COOR⁶; COR⁶; CONR⁵R⁶; CN; O=S(-R⁶)=O; O=S(-OR⁶)=O; R⁷O-P-(-OR⁸)=O; eine C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; eine C₆₋₁₈-Arylgruppe, die gegebenenfalls substituiert ist; eine C₃₋₇-Heteroarylgruppe, die gegebenenfalls substituiert ist;
- R⁵ bis R⁸, die gleich oder verschieden sind, Wasserstoff; eine C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Bicycloalkenylgruppe; eine C₇₋₁₀-Cycloalkenylgruppe; eine Arylgruppe, die gegebenenfalls substituiert ist; eine Heteroarylgruppe, die gegebenenfalls substituiert ist;
- n 1 bis 3;
wobei die Gruppen R³ bis R⁸ mit den Kohlenstoffatomen, an die sie gebunden sind, einen C₅₋₆-Ring bilden können, der kondensiert sein kann.

19. Emulsion nach Anspruch 18, wobei die Verbindung der Formel (I) unter den Verbindungen ausgewählt ist, für die bedeuten:
- n = 1 oder 2;
- R¹ und R², die gleich oder verschieden sind, Wasserstoff, eine C₁₋₂₀-Alkylgruppe; eine C₁₋₁₂-Alkoxygruppe; eine C₁₋₁₂-Monoalkylaminogruppe, eine C₁₋₁₂-Dialkylaminogruppe; einen Wasserlöslichkeit vermittelnden Substituenten, der unter Carboxylat, Sulfonat oder Ammonium ausgewählt ist;
- R³ eine Gruppe COOR⁵; COR⁵; CONR⁵R⁶; eine C₁₋₂₀-Alkylgruppe; eine C₃₋₁₀-Cycloalkylgruppe; eine C₃₋₁₀-Cycloalkenylgruppe; eine C₇₋₁₀-Bicycloalkylgruppe; Phenyl, Naphthyl oder Thienyl, die gegebenenfalls substituiert sind;
- R⁴ eine Gruppe COOR⁶; COR⁶; CONR⁵R⁶; eine C₁₋₂₀-Alkylgruppe; eine C₃₋₆-Cycloalkylgruppe; eine C₃₋₁₀-Cycloalkenylgruppe; eine C₇₋₁₀-Bicycloalkylgruppe; Phenyl, Naphthyl oder Thienyl, die gegebenenfalls substituiert sind;
- R⁵ und R⁶, die gleich oder verschieden sind, Wasserstoff; eine C₁₋₁₂-Alkylgruppe; eine C₃₋₁₀-Cycloalkylgruppe; eine C₃₋₁₀-Cycloalkenylgruppe; eine C₇₋₁₀-Bicycloalkylgruppe; eine C₃₋₁₀-Bicycloalkenylgruppe; Phenyl oder Naphthyl, die gegebenenfalls substituiert sind.

20. Emulsion nach Anspruch 19, wobei die Verbindung der Formel (I) unter den Verbindungen ausgewählt ist, für die bedeuten:
- R¹ und R², die gleich oder verschieden sind, Wasserstoff, eine C₁₋₂₀-Alkylgruppe; eine C₁₋₂₀-Alkoxygruppe; einen Wasserlöslichkeit vermittelnden Substituenten, der unter Carboxylat, Sulfonat oder Ammonium ausgewählt ist;
- R³ eine Gruppe COOR⁵; COR⁵; CONR⁵R⁶;
- R⁴ eine Gruppe COOR⁶; COR⁶; CONR⁵R⁶;
- R⁵ und R⁶, die gleich oder verschieden sind, Wasserstoff; eine C₁₋₁₂-Alkylgruppe; eine C₃₋₆-Cycloalkylgruppe; eine C₃₋₁₀-Cycloalkenylgruppe; eine C₇₋₁₀-Bicycloalkylgruppe; eine C₃₋₁₀-Bicycloalkenylgruppe; Phenyl oder Naphthyl, die gegebenenfalls substituiert sind.

21. Emulsion nach Anspruch 20, wobei die Verbindung der Formel (I) unter den Verbindungen der folgenden Formel (I') ausgewählt ist: 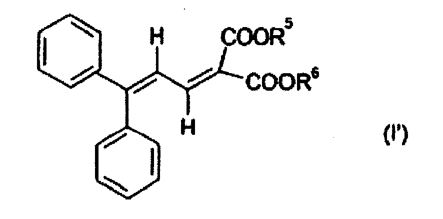 wobei R⁵ und R⁶, die gleich oder verschieden sind, Wasserstoff; eine C₁₋₂₀-Alkylgruppe; eine C₃₋₆-Cycloalkylgruppe; eine C₃₋₁₀-Cycloalkenylgruppe bedeuten.

22. Emulsion nach Anspruch 21, wobei die Verbindung der Formel (I') das 1,1-Dicarboxy-(2',2'-dimethyl-propyl)-4,4-diphenylbutadien der folgenden Struktur ist:

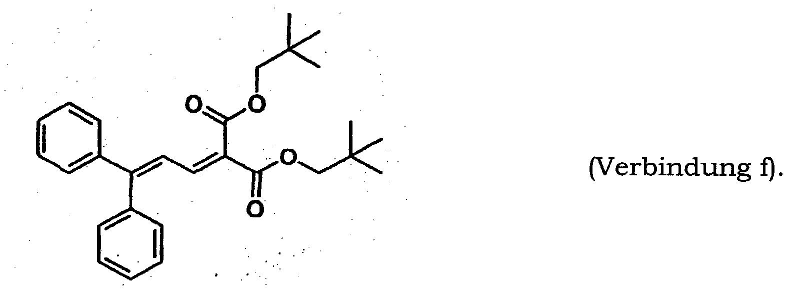

23. Emulsion nach einem der Ansprüche 1 bis 17, wobei der UV-A-Filter vom 4,4-Diarylbutadientyp der folgenden Formel (II) entspricht: 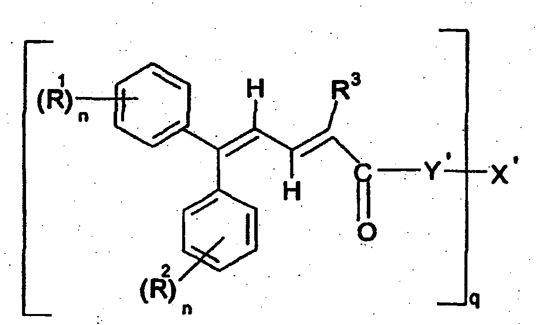 in der das Dien-System die Konfiguration Z,Z; Z,E; E,Z oder E,E aufweist oder Mischungen dieser Konfigurationen bedeutet und worin bedeuten:
- R¹, R², R³ und n die in Anspruch 18 für die Formel (I) angegebenen Bedeutungen;
- Y' eine Gruppe -O- oder -NR⁹-;
- R⁹ Wasserstoff; eine geradkettige oder verzweigte C₁₋₂₀-Alkylgruppe; eine C₂₋₁₀-Alkenylgruppe; eine C₃₋₁₀-Cycloalkylgruppe; eine C₇₋₁₀-Bicycloalkylgruppe; eine C₃₋₁₀-Cycloalkenylgruppe; eine C₇₋₁₀-Bicycloalkenylgruppe; Aryl; Heteroaryl;
- X' einen geradkettigen oder verzweigten, aliphatischen oder cycloaliphatischen C₂₋₂₀-Polyolrest, der 2 bis 10 Hydroxygruppen und die Valenz q aufweist; wobei die Kohlenstoffkette dieses Restes unterbrochen sein kann durch ein oder mehrere Schwefel- oder Sauerstoffatome; eine oder mehrere Imingruppen; eine oder mehrere C₁₋₄-Alkyliminogruppen;
- q 2 bis 10.

24. Emulsion nach Anspruch 23, wobei die Verbindung der Formel (II) unter den Verbindungen ausgewählt ist, für die bedeuten:
- R¹ und R², die gleich oder verschieden sind, Wasserstoff, eine C₁₋₁₂-Alkylgruppe; eine C₁₋₈-Alkoxygruppe; einen Wasserlöslichkeit vermittelnden Substituenten, der unter Carboxylat, Sulfonat oder Ammonium ausgewählt ist;
- R³ eine Gruppe COOR⁵; CONR⁵R⁶; CN; eine C₃₋₁₀-Cycloalkylgruppe; eine C₇₋₁₀-Bicycloalkylgruppe;
- R⁵ und R⁶, die gleich oder verschieden sind, eine geradkettige oder verzweigte C₁₋₂₀-Alkylgruppe; eine C₃₋₁₀-Cycloalkylgruppe; eine C₇₋₁₀-Bicycloalkylgruppe; Naphthyl oder Phenyl, die gegebenenfalls substituiert sind;
- X' einen C₂₋₂₀-Polyolrest, der 2 bis 6 Hydroxygruppen und insbesondere 2 bis 4 Hydroxygruppen enthält.

25. Emulsion nach Anspruch 24, wobei die Verbindung der Formel (II) unter den Verbindungen ausgewählt ist, für die X' einen Pentaerythritolrest bedeutet.

26. Emulsion nach Anspruch 25, wobei die Verbindung der Formel (II) unter den folgenden Verbindungen ausgewählt ist:

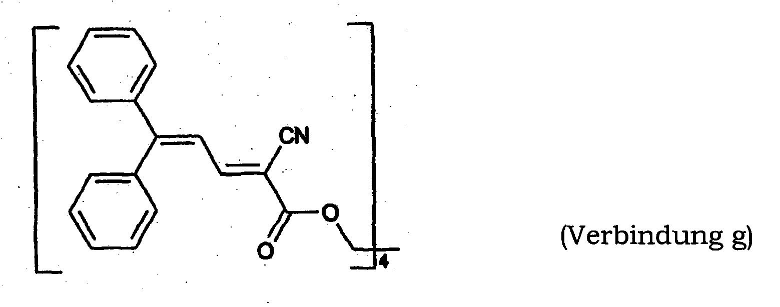

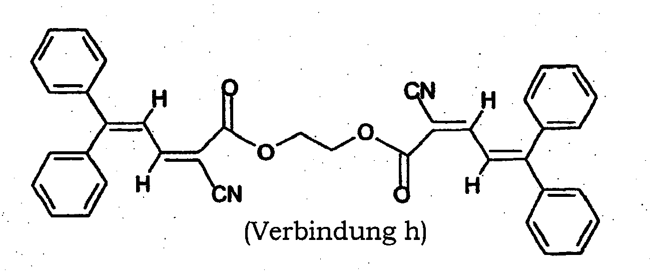

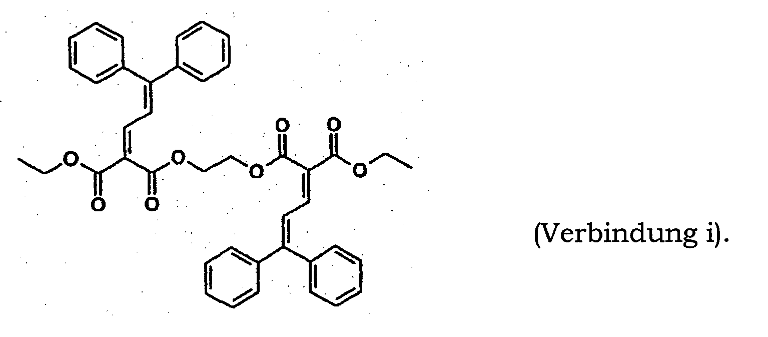

27. Emulsion nach einem der Ansprüche 1 bis 16, **dadurch gekennzeichnet, dass** das oder die 4,4-Diarylbutadien(e) in einem Anteil von 0,1 bis 20 Gew.-% und vorzugsweise 1 bis 10 Gew.-%, bezogen auf das Gesamtgewicht der Emulsion, enthalten sind.

28. Emulsion nach einem der Ansprüche 1 bis 27, **dadurch gekennzeichnet, dass** sie darüber hinaus mindestens einen ergänzenden, organischen oder anorganischen Sonnenschutzfilter umfasst, der im UV-A- und/oder UV-B-Bereich wirksam und wasserlöslich, fettlöslich oder in den üblicherweise verwendeten kosmetischen Lösungsmitteln unlöslich ist.

29. Emulsion nach Anspruch 28, wobei die ergänzenden organischen Filter unter den Anthranilaten; Zimtsäurederivaten; Dibenzoylmethanderivaten; Salicylsäurederivaten, Campherderivaten; Triazinderivaten; Benzophenonderivaten; β,β-Diphenylacrylatderivaten; Benzotriazolderivaten; Benzalmalonatderivaten; Benzimidazolderivaten; Imidazolinen; Bis-benzoazolylderivaten; Derivaten von p-Aminobenzoesäure (PABA); Benzoxazolderivaten; Derivaten von Methylen-bis(hydroxyphenyl-benzotriazol); Polymerfiltern und Siliconfiltern; Dimeren, die von α-Alkylstyrol abgeleitet sind, und deren Gemischen ausgewählt sind.

30. Emulsion nach Anspruch 29, wobei die ergänzenden organischen Filter ausgewählt sind unter:
Ethylhexyl Salicylate,
Ethylhexyl Methoxycinnamate,
Octocrylene,
Butyl Methoxydibenzoylmethane,
Phenylbenzimidazole Sulfonic Acid,
Benzophenone-3,
Benzophenone-4,
Benzophenone-5,
n-Hexyl-2-(4-diethylamino-2-hydroxybenzoyl)-benzoat,
4-Methylbenzylidene Camphor,
Terephthalylidene Dicamphor Sulfonic Acid,
Disodium Phenyl Dibenzimidazole Tetra-sulfonate,
2,4,6-Tris-(diisobutyl-4'amino-benzalmalonat)-s-triazin,
Anisotriazine,
Ethylhexyl Triazone,
Diethylhexyl Butamido Triazone,
Methylene bis-Benzotriazolyl Tetramethylbutylphenol,
Drometrizole Trisiloxane,
Polysilicone-15,
2,4-Bis[5-1-(dimethylpropyl)benzoxazol-2-yl-(4-phenyl)-imino]-6-(2-ethylhexyl)-imino-1,3,5-triazin und ihren Gemischen.

31. Zusammensetzung nach Anspruch 28, wobei die anorganischen ergänzenden Filter unter den gegebenenfalls umhüllten Pigmenten oder Nanopigmenten von Metalloxiden ausgewählt sind.

32. Zusammensetzung nach Anspruch 31, in der die ergänzenden anorganischen Filter Nanopigmente von amorphem oder kristallinem Titanoxid in Form von Rutil und/oder Anatas, Eisenoxid, Zinkoxid, Zirconiumoxid oder Ceroxid sind.

33. Emulsion nach einem der Ansprüche 1 bis 32, **dadurch gekennzeichnet, dass** sie ferner mindestens ein Bräunungsmittel und/oder Mittel zur künstlichen Braunfärbung der Haut enthält.

34. Emulsion nach einem der Ansprüche 1 bis 33, **dadurch gekennzeichnet, dass** sie ferner mindestens einen kosmetischen Zusatzstoff enthält, der unter den organischen Lösungsmitteln, ionischen oder nichtionischen Verdickungsmitteln und/oder Gelbildnern, beruhigenden Stoffen, Feuchthaltemitteln, Trübungsmitteln, Stabilisatoren, Emollientien, Siliconen, insektenabwehrenden Wirkstoffen, Parfums, Konservierungsmitteln, grenzflächenaktiven Stoffen, Füllstoffen, Pigmenten, Polymeren, Treibmitteln, Alkalisierungsmitteln oder Ansäuerungsmitteln oder beliebigen anderen Bestandteilen, die gewöhnlich auf dem Gebiet der Kosmetik und/oder Dermatologie verwendet werden, ausgewählt ist.

35. Emulsion nach einem der Ansprüche 1 bis 34, **dadurch gekennzeichnet, dass** sie, bezogen auf das Gesamtgewicht der Zusammensetzung, höchstens 1 Gew.-% eines emulgierenden grenzflächenaktiven Stoffes und sogar gar keinen emulgierenden grenzflächenaktiven Stoff enthält.

36. Emulsion nach einem der Ansprüche 1 bis 34, **dadurch gekennzeichnet, dass** sie ferner mindestens einen Gelbildner enthält.

37. Emulsion nach Anspruch 36, wobei der oder die Gelbildner in Konzentrationen von 0,1 bis 10 %, vorzugsweise 0,1 bis 5 % und besser 0,1 bis 3 % des Gesamtgewichts der Emulsion verwendet werden.

38. Emulsion nach Anspruch 36 oder 37, wobei der Gelbildner unter den nichtionischen oder anionischen, in Wasser löslichen oder in Wasser dispergierbaren assoziativen Polymeren ausgewählt ist, die mindestens eine hydrophobe Sequenz und mindestens eine hydrophile Sequenz enthalten.

39. Emulsion nach Anspruch 36 oder 37, wobei die nichtionischen assoziativen Polymere ausgewählt sind unter:
(1) Cellulosen, die mit Gruppen modifiziert sind, die mindestens eine hydrophobe Kette aufweisen;
(2) Hydroxypropylguarverbindungen, die mit Gruppen modifiziert sind, die mindestens eine Fettkette mit 10 bis 30 Kohlenstoffatomen aufweisen;
(3) Urethanpolyether, die in ihrer Kette gleichzeitig hydrophile Sequenzen, die meistens vom polyethoxylierten Typ sind, und hydrophobe Sequenzen enthalten, die aliphatische Verknüpfungen alleine und/oder cycloaliphatische und/oder aromatische Verknüpfungen sein können;
(4) Copolymere von Vinylpyrrolidon und hydrophoben Monomeren mit Fettkette;
(5) Copolymeren von Alkyl(C₁₋₆)acrylaten oder Alkyl(C₁₋₆)methacrylaten und amphiphilen Monomeren, die mindestens eine Fettkette enthalten;
(6) Copolymeren von hydrophilen Methacrylaten oder Acrylaten und hydrophoben Monomeren, die mindestens eine Fettkette enthalten.

40. Emulsion nach Anspruch 38, wobei die anionischen assoziativen Polymere unter den Copolymeren von Acrylsäure oder Methacrylsäure ausgewählt sind, die eine hydrophile Sequenz und mindestens eine hydrophobe Sequenz enthalten.

41. Emulsion nach Anspruch 38, wobei die anionischen assoziativen Polymere ausgewählt sind unter:
- dem Terpolymer Acrylsäure/Ethylacrylat/Alkylacrylat;
- dem Copolymer Acrylates/Steareth-20 Methacrylate;
- dem ethoxylierten (25 EO) Terpolymer (Meth)acrylsäure/Ethylacrylat/Behenylacrylat;
- dem ethoxylierten (20 EO) Copolymer Acrylsäure/Monocetylitaconat;
- dem ethoxylierten (20 EO) Copolymer Acrylsäure/Monostearylitaconat;
- dem Copolymer Acrylate/ mit polyethoxylierten (25 EO) C₁₂₋₂₄-Alkoholen modifiziertem Acrylat;
- dem Terpolymer Methacrylsäure/Methylacrylat/Dimethylmetaisopropenylbenzylisocyanat von ethoxyliertem Behenylalkohol.

42. Emulsion nach Anspruch 36 oder 37, wobei der Gelbildner unter den in Wasser löslichen Polymeren ausgewählt ist, die keine hydrophobe Kette enthalten.

43. Emulsion nach Anspruch 42, wobei die wasserlöslichen Polymere, die keine hydrophobe Kette aufweisen, unter den Homopolymeren und Copolymeren von Ethylenoxid; Polyvinylalkoholen; Homopolymeren und Copolymeren von Vinylpyrrolidon; Homopolymeren und Copolymeren von Vinylcaprolactam; Homopolymeren und Copolymeren von Polyvinylmethylether; nichtionischen Acrylhomopolymeren und Acrylcopolymeren; Alkyl-C₁-C₂-cellulosen und ihren Derivaten; Hydroxymethylpropylcellulose; Alkyl-C₁-C₃-guarverbindungen oder Hydroxyalkyl-C₁-C₃-guarverbindungen ausgewählt sind.

44. Emulsion nach Anspruch 36 oder 37, wobei der Gelbildner unter den Kieselsäuren mit einer Größe im Nanometerbereich, Fettalkoholen, Algenderivaten, natürlichen Gummen wie Traganth, Tonen, Polysacchariden und synthetischen Polymeren ausgewählt ist.

45. Verfahren zur Herstellung einer Emulsion, wie sie in einem der Ansprüche 1 bis 44 definiert ist, das darin besteht, in einem ersten Schritt die wässrige Phase, die Ölphase und die Polymerpartikel unter Rühren zu vermischen und dann in einem zweiten Schritt das erhaltene Gemisch einer auf dem Prinzip der Kavitation beruhenden Homogenisierung zu unterziehen.

46. Verfahren zur Herstellung nach Anspruch 45, **dadurch gekennzeichnet, dass** in dem zweiten Schritt die Homogenisierung bei einem Druck von 400 bis 700 bar durchgeführt wird.

47. Verwendung einer Zusammensetzung wie sie in einem der Ansprüche 1 bis 43 definiert ist, für die Herstellung von Produkten zur kosmetischen Behandlung der Haut, der Lippen und der Haare einschließlich der Kopfhaut, insbesondere für den Schutz und/oder die Pflege Haut, der Lippen und/oder der Haare und/oder zum Schminken der Haut und/oder der Lippen.
